# FASCICULE DE BREVET EUROPEEN

(11) **EP 3 475 913 B1**
(45) Date de publication et mention de la délivrance du brevet: **18.11.2020**
(21) Numéro de dépôt: 17814384.8
(22) Date de dépôt: 22.06.2017
(51) Int. Cl.: G06Q 50/22, A61J 7/00, G01G 19/387, A61J 7/02, G16H 20/13, B65B 57/00

(54) **SYSTÈMES ET MÉTHODES D'IDENTIFICATION DE MÉDICAMENTS DÉPOSÉS DANS UNE CASE D'UN PILULIER SELON UNE PRESCRIPTION**
SYSTEME UND VERFAHREN ZUR IDENTIFIZIERUNG VON IN EINEM FACH EINER PILLENSCHACHTEL DEPONIERTEN MEDIKAMENTEN GEMÄSS EINER VERSCHREIBUNG
SYSTEMS AND METHODS FOR IDENTIFYING MEDICINES DEPOSITED IN A COMPARTMENT OF A PILL BOX ACCORDING TO A PRESCRIPTION

(30) Priorité: 23.06.2016 CA 2933860
(43) Date de publication de la demande: 01.05.2019
(73) Titulaire: Grosfils, Matthieu, Montréal, Québec H3X 2Z2 (CA)
(72) Inventeur: FORTÉ, Jean-Pierre, Longueuil, Québec J4G 2A8 (CA); GROSFILS, Matthieu, Montréal, Québec H3X 2Z2 (CA)
(74) Mandataire: Regimbeau
(86) Numéro de dépôt international: PCT/CA2017/050763
(87) Numéro de publication internationale: WO 2017/219144

(56) Documents cités:
- WO-A1-2015/126254
- WO-A1-2015/170762
- WO-A2-02/25568
- CA-A1- 2 217 220
- US-A- 2 940 355
- US-A- 4 812 904
- US-A- 5 150 425
- US-A- 5 806 670
- US-A- 6 026 189
- US-A1- 2006 045 323
- US-A1- 2009 080 735
- US-A1- 2010 284 607
- US-A1- 2011 081 087
- US-A1- 2012 083 666
- US-A1- 2012 201 434
- US-A1- 2013 142 406
- US-A1- 2014 113 283
- US-A1- 2015 066 204
- US-A1- 2015 127 145
- US-A1- 2015 302 255
- US-A1- 2015 331 887
- US-A1- 2015 350 570
- US-A1- 2016 007 138
- US-A1- 2016 073 096
- US-A1- 2016 085 940
- US-A1- 2016 132 660
- US-B1- 6 324 253
- US-B1- 6 324 253
- US-B1- 6 535 637
- US-B1- 6 535 637
- US-B1- 9 336 552
- US-B2- 7 080 755
- US-B2- 7 218 395
- US-B2- 7 828 147
- US-B2- 7 874 489
- US-B2- 8 374 965
- US-B2- 8 600 548
- US-B2- 8 727 208
- US-B2- 8 914 146
- US-B2- 9 238 518
- US-B2- 9 238 518
- US-B2- 9 299 212
- US-B2- 9 313 464
- US-B2- 9 345 636

## Description

### DOMAINE DE LA DIVULGATION

La présente divulgation est en lien avec les méthodes et appareillages pour réduire les erreurs médicales, et plus spécifiquement lors de l'identification des médicaments par leurs caractéristiques externes.

La présente divulgation est relative à des systèmes et équipements permettant l'identification et la vérification de médicaments (comprimé/gélule/capsule et autres formes sèches) préalablement disposés dans les alvéoles/ cases d'un distributeur de médicaments (carte alvéolée/blister-card/pilulier/semainier/dosette/ pill dispenser).

La présente divulgation concerne également les procédés de fabrication de ces systèmes et équipements ainsi que les méthodes d'utilisation de ces systèmes et équipements.

### ART ANTÉRIEUR

Dans les pharmacies, les pharmaciens délivrent aux patients les médicaments spécifiés par les prescriptions réalisées par les médecins. L'administration des prescriptions est généralement organisée selon quatre moments de la journée matin (déjeuner), midi (dîner), soir (souper) et couché.

Les médicaments sont fournis par les grossistes, les laboratoires pharmaceutiques dans des conditionnements de grande taille. Les assistants techniques en pharmacie préparent les quantités de chaque médicament en fonction du plan de prise validé par le pharmacien et selon la prescription du médecin. Pour chaque semaine, les médicaments sont placés dans les cases du blister, plusieurs médicaments peuvent être réunis dans la même case représentant un temps de prise donné. La préparation des médicaments dans leur conditionnement final est une étape qui nécessite de large quantités de main d'œuvre, qui peut être source d'erreur et peut résulter dans des erreurs de dosage et/ou d'erreur de type de médicament finalement présent dans le blister.

Une fois que le blister a été préparé par l'assistant technique en pharmacie selon le plan de prise validé par le pharmacien, celui-ci est transmis au pharmacien pour vérification. Le pharmacien contrôle donc le contenu de chaque case en comparant par rapport au plan de prise. Pour déterminer l'adéquation entre le médicament présent et le besoin, il fait appel à sa mémoire et si cela n'est pas possible il compare le médicament présent dans le blister avec une photo qu'il affiche sur un écran d'ordinateur. Cette phase demande beaucoup de temps et d'attention de la part du pharmacien et présente des risques d'erreur en cas de la présence concomitante de médicaments dont les caractéristiques physiques sont similaires. Cette vérification est souvent réalisée avec de fortes contraintes temporelles, car le pharmacien est en bout de chaîne et il est le seul habilité à libérer les prescriptions. Le pharmacien approuve le contenu du blister en scellant une feuille adhésive par-dessus ce qui interdit toute modification future du contenu du blister.

Les médicaments conditionnés dans le blister sont délivrés au patient. La prescription du médecin est archivée à la pharmacie. Le patient ou l'aidant n'a donc plus de possibilité de vérifier le contenu du blister par rapport à la prescription.

D'après les données de Stat Ramq, au Québec plus de 16 300 000 médicaments sont délivrés chaque année après une mise en blister. D'après To Err Is Human Building a Safer Health System NATIONAL ACADEMY PRESS Washington, D.C. 1999, aux États-Unis, des estimations montrent qu'en moyenne 20 personnes meurent chaque jour suite à des erreurs de délivrance dans les hôpitaux. 1,5 million de personnes par an sont atteintes par des erreurs de délivrance et le coût direct et indirect de ce type d'erreur est de 15 à 20 milliards de USD par an en Europe et aux EU. Garantir que chaque patient reçoit le bon dosage à chaque période peut s'avérer difficile et mener à des erreurs.

La demande internationale de brevet WO 2015/126254 ainsi que la demande US 2015/066204 décrivent un équipement et une méthode pour vérifier des médicaments en dose individuelle et avant leur positionnement dans un compartiment.

La demande de brevet publiée sous le numéro US 2015/0350570 décrit une méthode pour le scannage en surface d'un médicament.

La demande de brevet publiée sous le numéro US 2013/0142406 décrit une méthode et un appareil pour la vérification d'une prescription médicamenteuse.

La demande de brevet publiée sous le numéro US 2012/0290129 décrit un système et une méthode de vérification avant de placer un médicament dans un compartiment.

La demande de brevet publiée sous le numéro US 2012/0201434 décrit un système et une méthode pour l'inspection de médicaments empaquetés par comparaison d'images.

La demande de brevet publiée sous le numéro US 2006/0045323 décrit un système de comptage et de vérification d'objets qui ne se superposent pas.

Il existait un besoin pour un solution technologique dépourvue d'au moins un des inconvénients des équipements et méthodes de l'art antérieur.

Il existait donc aussi un besoin pour des méthodes de fabrication des équipements simples, fiables et dépourvues d'au moins un des inconvénients des procédés de l'art antérieur.

Il existait donc également un besoin pour une solution technologique dépourvu d'au moins un des inconvénients suivants:
- manque de fiabilité ou fiabilité incomplète;
- temps d'opération élevé entraînant un cout important du contrôle; et
- inapplicable en cas de panne de courant.

### SOMMAIRE DE LA DIVULGATION

Les aspects de la demande sont définies dans les revendications ci-jointes.

### BRÈVE DESCRIPTION DES FIGURES

Les exemples présentés dans cette section des figures sont non-limitatifs et son présentés dans le but d'exemplifier différentes variantes de la technologie de la présente divulgation en illustrant de telles variantes.
Figure 1 représente en vue de face dans un mode avantageux de réalisation, la structure semi fermée d'un système de la présente divulgation comportant un dispositif d'imagerie comprenant au moins une caméra au niveau supérieur et dirigée vers la surface supérieure du compartiment et au moins une caméra au niveau inférieur dirigé vers la surface inférieure du compartiment, un support horizontal et un dispositif d'éclairage comportant 4 lumières positionnées dans les coins de la structure.
Figure 2 représente en vue de côté et en coupe horizontale un dispositif d'identification des médicaments présents dans les compartiments d'un distributeur de médicament, sur la base de leurs caractéristiques (géométriques et/ou autre) obtenues à partir d'un dispositif d'imagerie.
Figure 3A représente une vue en perspective d'un plateau de préparation.
Figure 3B représente une vue en perspective du plateau de préparation avec le tiroir dans une position rétractée.
Figure 4A représente une vue en perspective d'un plateau de préparation.
Figure 4B représente une vue en perspective du plateau de préparation dans une position inversée.
Figure 5A représente une vue en perspective du pilulier.
Figure 5B représente une vue en perspective du pilulier dans une position inversée.
Figure 6A représente en vue en perspective supérieure un système à étages de la présente divulgation, le distributeur surdimensionné étant rempli de médicament selon une ordonnance et positionné au-dessus d'un distributeur standard; le transfert des médicaments du distributeur surdimensionné vers le distributeur standard se faisant pas un faisceau de conduits de connexion sensiblement parallèle et placé à la verticale du lieu.
Figure 6B: représente en vue en perspective supérieure le distributeur standard après que les médicaments présents dans le distributeur supérieur lui aient été transférés.
Figure 6C représente une vue en perspective du système à étage.
Figure 6D représente une vue en perspective du système d'identification de médicaments avec le tiroir dans une position rétractée.
   inversée.
Figure 7A représente une vue en perspective d'un dispositif de connexion.
Figure 7B représente une vue en perspective du dispositif de connexion dans une position renversée.
Figure 8A représente une vue en perspective d'un dispositif de connexion.
Figure 8B représente une vue en perspective du dispositif de connexion dans une position renversée.
Figure 9 décrit une méthode d'indentification de médicaments selon un aspect de la divulgation.

### DESCRIPTION DÉTAILLÉE DE LA PRÉSENTE DIVULGATION

Les exemples présentés dans cette section sont non-limitatifs et présentés dans le but d'exemplifier différentes variantes de la technologie de la présente divulgation.

### DÉFINITIONS PRÉLIMINAIRES

*Médicament:* dans son sens large applicable à la généralité de la présente divulgation, il s'agit de tout item qu'il faut placer dans un distributeur muni de compartiments et selon des instructions de distribution dans les compartiments dudit distributeur.

*Prescription ou ordonnance:* signifie toute instruction de distribution d'items dans des compartiments d'un distributeur.

Le système d'identification comprend un dispositif qui est configuré pour analyser les caractéristiques d'identification des médicaments disposés dans le blister : forme, caractéristiques géométriques, taille, couleur, dimensions, texture, marquages. Les caractéristiques de chacun des médicaments du blister sont isolées puis comparées avec les informations contenues dans la base de données ce qui permet leur identification. Le détail du contenu identifié du blister est comparé avec les informations de prescription. Le blister est aussi appelé un pilulier ou une carte alvéolée.

Selon une configuration de la méthode d'indentification de médicaments, le pilulier est flexible.

Selon une autre configuration de la méthode d'indentification de médicaments, la case est transparente.

Selon une configuration, la méthode d'indentification de médicaments comprend :
identifier, à partir du haut, au moins un premier médicament déposé dans une case d'un pilulier et analyser au moins deux caractéristiques d'identification dudit au moins un premier médicament;
identifier, à partir du bas, au moins un deuxième médicament déposé dans ladite case dudit pilulier et analyser au moins deux caractéristiques d'identification dudit au moins un deuxième médicament.

Par exemple, les caractéristiques d'identification sont choisies parmi une forme, une taille, une couleur, une dimension, une texture et un marquage.

Par exemple, le premier médicament est identifié par le biais d'une caméra.

Par exemple, le deuxième médicament est identifié par le biais d'une caméra.

Par exemple, le premier médicament et le deuxième médicament peuvent être identiques.

Par exemple, le premier médicament et le deuxième médicament sont différents.

Selon une configuration du système d'identification de médicaments, le premier dispositif de mesure comprend une caméra pour capter une première image de la case.

Par exemple, le deuxième dispositif de mesure comprend une caméra pour capter une deuxième image de la case.

Le système d'identification peut comprendre :
un premier dispositif de mesure pour identifier, à partir du haut, au moins un premier médicament déposé dans une case d'un pilulier et analyser au moins deux caractéristiques d'identification du premier médicament; et
un deuxième dispositif de mesure pour identifier, à partir du bas, au moins un deuxième médicament déposé dans ladite case dudit pilulier et analyser au moins deux caractéristiques d'identification du deuxième médicament.

Les caractéristiques d'identification sont choisies parmi une forme, une taille, une couleur, une dimension, une texture et un marquage.

Par exemple, le pilulier est flexible. La case peut être transparente.

Le premier médicament déposé peut être identifié par le biais d'une caméra. Le deuxième médicament déposé peut être identifié par le biais d'une caméra.

Par exemple, le premier médicament et le deuxième médicament peuvent être identiques. Selon une autre configuration, le premier médicament et le deuxième médicament peuvent être différents.

Une enceinte peut recevoir le système d'identification de médicaments. L'enceinte peut être flexible.

Par exemple, la méthode d'indentification de médicaments comprend l'activation d'un tiroir rétractable permettant au médicament le passage, par gravité, du compartiment vers le pilulier. La méthode peut comprendre une étape pour associer la prescription au compartiment du plateau.

La méthode peut comprendre une étape pour mesurer et contrôler un poids du compartiment.

Par exemple, la méthode d'indentification de médicaments comprend identifier un médicament déposé dans le compartiment et analyser au moins deux caractéristiques du médicament. Les caractéristiques du médicament peuvent être choisies parmi une forme, une taille, une couleur, une dimension, une texture et un marquage.

Par exemple, le système d'identification de médicaments a un dispositif de connexion qui comprend un tiroir rétractable. Le dispositif de connexion peut inclure un prisme trapézoidale muni de divers couloirs permettant de relier les compartiments avec les cases. Le dispositif de connexion peut inclure une base supérieure et une base inférieure, les bases étant reliées par le biais d'une pluralité de couloir permettant le passage des compartiments vers les cases. La surface inférieure du compartiment peut être supérieure à la surface inférieure de la case du pilulier. Le volume interne défini par le compartiment peut être supérieur au volume interne défini par la case du pilulier.

Par exemple, le système d'identification de médicaments a un dispositif d'identification permettant d'identifier au moins un médicament et analyser au moins deux caractéristiques d'identification d'au moins un médicament. Les caractéristiques du médicament sont choisies parmi une forme, une taille, une couleur, une dimension, une texture et un marquage. Un kit peut comprendre le système d'identification de médicaments et un pilulier.

Un premier aspect de la présente divulgation comprend des systèmes d'identification et de validation de médicaments disposés, selon une prescription, dans les compartiments d'un distributeur comprenant n compartiments, n'étant un nombre entier supérieur ou égal à 1, chacun des médicaments étant susceptible d'être au moins partiellement superposé avec au moins un autre médicament présent dans le même compartiment du distributeur, ledit système comprenant:
- un dispositif de mesure configuré pour recueillir des caractéristiques d'identification des médicaments disposés dans chacun des compartiments du distributeur;
- un dispositif de transmission configuré pour transférer, à une unité de calcul, les caractéristiques d'identification recueillies par le dispositif de mesure;
- une unité de calcul configurée pour déterminer à partir des caractéristiques d'identification recueillies si
- chacun des médicaments effectivement présents dans chacun des compartiments correspond ou non à un médicament qui, selon la prescription, devrait s'y trouver et/ou pour indiquer dans quel compartiment se trouve la ou les non-correspondances et/ou quelle est la nature de la ou des non-correspondances.

Les procédés de fabrication des systèmes de la présente divulgation, notamment ceux mettant en œuvre au moins une des techniques connues d'assemblage tels que notamment: le vissage, le collage, le boulonnage, l'emboîtage, le rivetage et la soudure, font également partie du premier objet de la présente divulgation .

Les utilisations du système d'identification et de validation de médicaments, tel que précédemment défini ou tels qu'obtenus par les procédés de fabrication précédemment définis, pour réduire le risque d'erreur lors de remplissage d'un distributeur de médicament selon une prescription, font également partie du premier aspect de la présente divulgation .

Les méthodes de validation de la conformité, à une prescription médicale, du remplissage d'un distributeur de médicament, et comprenant les étapes suivantes:
- de remplissage, selon une prescription médicale, des compartiments d'un distributeur, par un opérateur et/ou par un automate;
- de détermination, grâce à la mise en œuvre d'au moins un des systèmes d'identification selon la présente divulgation du contenu de chacun des compartiments; et
- de correction éventuelle du contenu d'un ou de plusieurs compartiments(s) du distributeur en cas de non-conformité constatée par ledit système par rapport à la prescription médicale,
   font également partie du premier aspect de la présente divulgation .

Un deuxième aspect de la présente divulgation comprend des systèmes à étages d'identification et de validation de médicaments disposés, selon une prescription, dans les compartiments d'un distributeur destiné à un patient et/ou destiné à un accompagnateur du patient, lesdits médicaments étant susceptibles d'être au moins partiellement superposés avec au moins un autre médicament présent dans le même compartiment du distributeur, ledit système comprenant:
- un premier distributeur destiné au patient ou à un accompagnateur du patient ;
- un deuxième distributeur surdimensionné (par rapport a la taille du premier distributeur) comportant le même nombre de compartiments ou un nombre de compartiments supérieur au nombre de compartiments du premier distributeur, ledit deuxième distributeur étant, de préférence d'une taille suffisante pour éviter le recouvrement de médicaments, et de préférence, positionné à une hauteur plus grande que le premier distributeur, et avantageusement, au-dessus ou décalé;
- un dispositif de connexion entre les compartiments du premier distributeur et ceux du deuxième distributeur, ledit dispositif de connexion permettant le transit, de préférence par gravité, d'un ou plusieurs médicaments depuis un compartiment du deuxième distributeur vers son vis-à-vis du premier distributeur, ledit dispositif de connexion étant de préférence constitué par un faisceau de conduits, de préférence sensiblement parallèle, chacun des conduits étant:
   - au niveau de sa partie supérieure solidarisé avec la partie inférieure d'un des compartiments du deuxième distributeur, et
      i. - au niveau de sa partie inférieure solidarisé avec la partie supérieure du compartiment du premier distributeur en vis-à-vis;
la partie inférieure d'un compartiment du deuxième distributeur surdimensionné étant configurée de façon a disposé d'un dispositif libérateur laissant, à la demande, les médicaments d'un des compartiments du deuxième distributeur migrer vers le compartiment en vis-à-vis du premier distributeur , et
l'ouverture supérieure d'un conduit du dispositif de connexion étant, de préférence, plus grande que l'orifice inférieur du conduit de connexion;
- un dispositif de mesure configuré pour analyser les caractéristiques d'identification des médicaments disposés de façon à ne pas se chevaucher dans chacun des compartiments du distributeur surdimensionné : qu'un des médicaments présents dans un compartiment soit séparé par un espace d'un ou plusieurs médicaments voisins ou qu'ils soit au contact avec un ou plusieurs médicament(s) adjacent(s) ;
- un dispositif de transmission configuré pour transférer les caractéristiques d'identification (analysées ou non analysées) recueillies par le dispositif de mesure et relatives aux médicaments, présents dans les compartiments du deuxième distributeur, à une unité de calcul;
- une unité de calcul configurée pour:
   - isoler les caractéristiques d'identification reçues du dispositif de transmission pour chacun des médicaments effectivement présents dans un compartiment du deuxième distributeur, et d'identifier chacun de ces médicaments,
   - comparer les caractéristiques isolées dans l'étape précédente avec les informations contenues dans une base de données des caractéristiques des médicaments devant, selon la prescription, être présents dans un des compartiments du distributeur, et
   - ledit système d'identification et de validation émettant un message selon lequel :
      i. - tous les médicaments effectivement présents dans chacun des compartiments du deuxième distributeur correspondent ou non à ceux qui selon la prescription devraient s'y trouver le nombre de médicament effectivement présent dans un compartiment correspond au nombre de médicaments qui devraient, selon la prescription, s'y trouver; et/ou
   - le nombre total de médicaments présents dans tous les compartiments du distributeur correspond au nombre total de médicaments qui devraient, selon la prescription, se trouver dans le distributeur; et/ou
   - le poids des médicaments présents dans un compartiment correspond au poids des médicaments qui devraient, selon la prescription, s'y trouver et/ou
- le poids de tous les médicaments présents dans tous les compartiments du distributeur correspond au poids total de tous les médicaments, qui selon la prescription, devraient s'y trouver.

Les procédés de fabrication des systèmes d'identification à étages, par mise en œuvre d'au moins une des techniques connues d'assemblage tels que notamment: le vissage, le collage, le boulonnage, l'emboîtage, le rivetage et la soudure, sont également parties de la présente divulgation.

Les utilisations des systèmes d'identification à étages pour réduire le risque d'erreur lors de remplissage d'un distributeur de médicament selon une prescription font également partie de la présente divulgation .

Les méthodes de validation de la conformité à une prescription médicale, du remplissage d'un distributeur de médicament comprenant les étapes suivantes:
- de remplissage des compartiments d'un distributeur surdimensionné selon une prescription médicale;
- de détermination du contenu de chacun des compartiments grâce à la mise en œuvre d'un système d'identification;
- de correction éventuelle du contenu d'un des compartiments du distributeur en cas de non-conformité constatée par rapport à la prescription médicale; et
- de transit des médicaments présents dans un compartiment du distributeur surdimensionné dans le compartiment vis à vis du premier distributeur, font également partie de la présente divulgation .

Un autre aspect de la présente divulgation comprend des équipements d'aide au contrôle de la conformité du contenu d'un distributeur de médicaments à une ordonnance, comportant:
- un premier et un deuxième distributeur de médicaments, le deuxième distributeur étant surdimensionné par rapport au premier distributeur;
- un dispositif de connexion entre les compartiments du premier distributeur et ceux du deuxième distributeur, ledit dispositif permettant le transit, de préférence par gravité, d'un ou plusieurs médicaments depuis un compartiment du deuxième distributeur vers son vis-à-vis du premier distributeur, ledit dispositif de connexion étant de préférence constitué par un faisceau de conduits, chacun des conduits du faisceau étant:
   - au niveau de sa partie supérieure solidarisé avec la partie inférieure d'un des compartiments du deuxième distributeur, et
      i. - au niveau de sa partie inférieure solidarisé avec la partie supérieure d'un compartiment du premier distributeur en vis-à-vis;
la partie inférieure d'un compartiment du distributeur surdimensionné étant configuré de façon a disposé d'un dispositif libérateur laissant, à la demande, les médicaments d'un des compartiments du deuxième distributeur migrer vers le compartiment en vis-à-vis du premier distributeur, et
l'ouverture supérieure d'un conduit de connexion étant de préférence plus grande que l'orifice inférieur du conduit de connexion.

Les procédés de fabrication d'un équipement d'aide au contrôle par mise en œuvre d'au moins une des techniques connues d'assemblage tels que notamment: le vissage, le collage, le boulonnage, l'emboîtage, le rivetage et la soudure, font également partie de la présente divulgation.

Les utilisations des équipements pour réduire le risque d'erreur lors de remplissage d'un distributeur de médicaments selon une prescription sont également partie de la présente divulgation.

Les méthodes de validation de la conformité à une prescription médicale, du remplissage d'un distributeur de médicament intégré dans un équipement d'aide au contrôle de la conformité, ladite méthode comprenant les étapes suivantes:
- de remplissage des compartiments d'un distributeur surdimensionné selon une prescription médicale;
- de détermination du contenu de chacun des compartiments du distributeur surdimensionné;
- de correction éventuelle du contenu d'un des compartiments du distributeur surdimensionné en cas de non-conformité constatée par rapport à la prescription médicale; et
- de transit des médicaments présents dans un compartiment du distributeur surdimensionné dans le compartiment du premier distributeur en vis-à-vis, sont également partie de la présente divulgation .

Le prototype de la présente divulgation permet de vérifier le contenu du blister contenant un ou plusieurs médicaments. La vérification peut se faire automatiquement. La vérification peut se faire sans contact humain. La vérification des caractéristiques des médicaments contenus dans le blister est réalisée selon une identification de chacune des caractéristiques des médicaments en utilisant des données bidimensionnelles et tridimensionnelles comme des données d'un "nuage de points", obtenus par différents systèmes et/ou méthodes.

Selon un prototype décrit, le système peut comprendre un dispositif d'imagerie configuré pour recueillir les données image de surface du contenu des cases du blister contenant un ou plusieurs médicaments, et un contrôleur configuré pour contrôler le dispositif d'imagerie pour le recueil des données image d'un ou plusieurs médicaments disposés dans les cases du blister.

Le système est configuré pour :
produire un nuage de points 3D à partir des données des images de surface d'un ou plusieurs médicaments contenus dans les cases du blister ;
produire des données géométriques pour chacun des médicaments à partir des nuages de point 3D ;
produire des données image bidimensionnelle pour chacun des médicaments
produire des données pour chacun des médicaments notamment les caractéristiques de leur aspect extérieur autre que géométrique comme, par exemple, leur couleur ;
identifier précisément le médicament correspondant à chacun des médicaments contenu dans les cases du blister en fonction des caractéristiques (géométriques et autres) produites par les données bidimensionnelles et tridimensionnelles (nuage de point 3D).

Selon un prototype décrit, un programme peut apporter les instructions à l'ordinateur pour réaliser les étapes d'identification des médicaments sur la base de leurs caractéristiques (géométriques et/ou autre) obtenues à partir d'un dispositif d'imagerie. La méthode comprend la réception des caractéristiques (géométriques et/ou autre) d'un médicament, ainsi que les caractéristiques produites à partir des données bidimensionnelles et tridimensionnelles (nuage de points 3D) d'un médicament. La méthode comprend également la détermination que les caractéristiques (géométriques et/ou autre) du ou des médicament(s) présent(s) dans les cases du blister correspondent aux caractéristiques (géométriques et/ou autre) d'un médicament connu.

La présente divulgation n'est pas limitée à une identification des médicaments par une reconnaissance optique seule, mais peut inclure d'autre méthode ou appareil pour compléter cette reconnaissance comme un laser, un spectromètre.

Les médicaments vérifiés inclus les composés suivants, mais ne sont pas limités aux :
- produits pharmaceutiques;
- nutraceutiques;
- vitamines;
- compléments alimentaires;
- gélules;
- capsules; et
- médicaments, avec prescription ou sans prescription.

Et tout autre médicament pouvant être emballé dans un emballage primaire, un emballage ou un contenant.

Méthodes d'analyse, d'inspection des médicaments contenus dans les cases d'un jeu de contenant individuel pour médicaments

Les informations d'identification obtenues pour les médicaments contenus dans les cases sont comparées avec les informations préalablement enregistrées dans la base de données pour chacun des médicaments prévues par la prescription. Une comparaison avec les caractéristiques des médicaments similaires est également effectuée.

Les caractéristiques des médicaments (taille, forme, volume, couleur, densité, texture, surface extérieure, marquage, luminescence...) présents dans les contenants individuels sont obtenues par des méthodes et équipements analytiques incluant, mais non limité(e)s à:
- capteur 2D lumière visible (photo ou vidéo);
- capteur 3D lumière visible;
- rayon X;
- décomposition RGB, analyse des couleurs avec illumination par des lumières de différentes couleurs incluant lumière blanche, lumière ultraviolette, lumière rouge, lumière verte, lumière bleue et/ou lumière infrarouge, lumière stroboscopique;
- reconnaissance optique des caractères (OCR) et/ou vérification optique des caractères (OCV);
- analyse spectroscopique (infrarouge proche, fluorescence);
- spectroscopie MMS (multimodal multiplex sampling), Raman;
- imagerie par résonnance magnétique;
- ultrasons;
- scanner par excitation laser;
- pesée de précision; et
   toute autre méthode analytique chimique et/ou physique.

Les exemples suivants sont donnés à titre illustratif seulement et ne seraient être interprétés comme constituant une quelconque limitation de la présente divulgation dans sa généralité.

### EXEMPLE 1 -Méthode avec boitier fermé

### Détail du système

Dans un configuration, le système de la présente divulgation comprend:
- une boitier contenant
- un support sur lequel est disposé un distributeur de médicament contenant les médicaments à vérifier,
- la taille du support est approximativement de21 x 21 cm;
- des dispositifs d'éclairages disposés dans les coins du boitier.
- des dispositifs d'imagerie permettant l'acquisition des données analysés par l'unité de calcul;
- un système de vérification par numérisation optique pour vérifier le contenu du plateau de préparation, le système optique peut être fixe ou mobile et se déplacer sur deux axes horizontaux afin de contrôler toute la surface des cellules du plateau; et une balance de précision peut être ajoutée pour contrôler le poids de l'ensemble des médicaments.

### Étapes de mise en œuvre de la méthode d'utilisation

1. Un blister distributeur de médicament rempli est disposé dans le support;
2. Les médicaments disposés sur le plateau sont comptés et vérifiés par un système de numérisation optique. Par exemple, on peut utiliser le système de numérisation optique comme décrit dans le système EyeCon® commercialisé par la société Avery Weigh-Tronix ;
3. Validation du contenu par le système de vérification par rapport à la prescription;
4. Retrait du blister distributeur de médicament par l'opérateur; et
5. Le blister est refermé par une feuille d'étanchéité par l'opérateur.

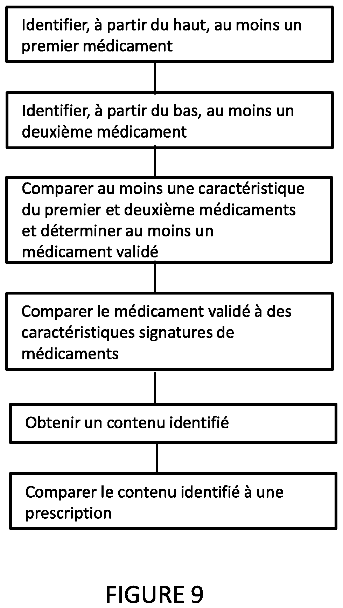

Selon un aspect de la présente demande, il est divulgué une méthode d'identification de médicaments. Se référant à la Figure 9, il est décrit une méthode d'indentification de médicaments. La méthode consiste à identifier, à partir du haut, au moins un premier médicament déposé dans une case d'un pilulier et analyser au moins une caractéristique d'identification du premier médicament. L'identification du premier médicament peut se faire à partir d'une image prise du haut du pilulier. La méthode consiste aussi à identifier, à partir du bas, au moins un deuxième médicament déposé dans la case du pilulier et analyser au moins une caractéristique d'identification du deuxième médicament. L'identification du deuxième médicament peut se faire à partir d'une image prise du bas du pilulier.

La méthode consiste à comparer au moins une caractéristique du premier médicament et au moins une caractéristique du deuxième médicament, pour déterminer au moins un médicament validé. La méthode consiste aussi à comparer au moins une caractéristique du médicament validé à des caractéristiques signatures de médicaments, pour obtenir un contenu identifié pour la case. La méthode consiste aussi à comparer le contenu identifié à une prescription.

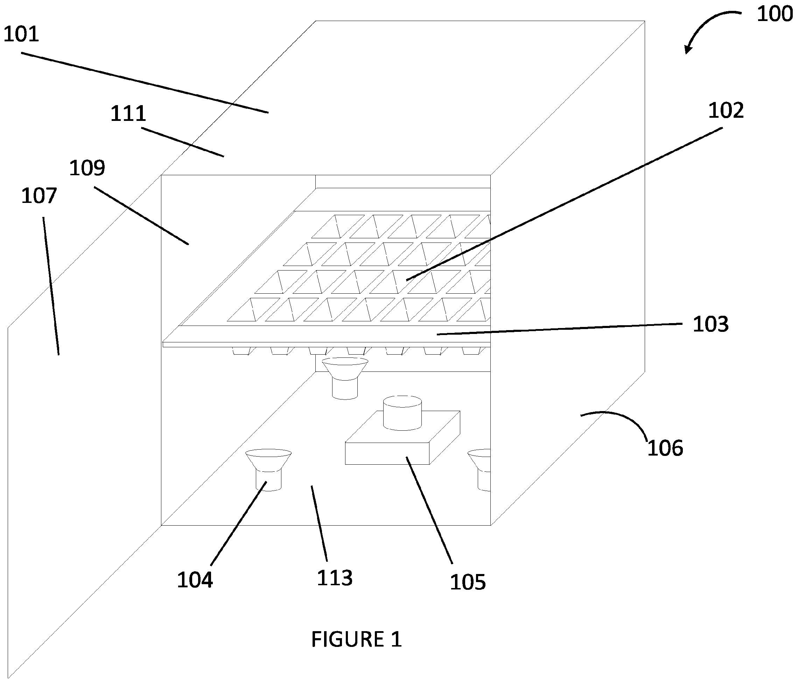

Selon un aspect de la présente demande, il est divulgué un système d'identification de médicaments. Un exemple de système d'identification 100 de médicaments est présenté à la Figure 1. Le système d'identification 100 inclut une enceinte 101. L'enceinte peut être un boitier ou une boite. La forme de l'enceinte peut être un prisme à base rectangulaire ou carrée. La base de l'enceinte peut avoir tout autre forme, par exemple une forme 3, 4, 5, 6, 7 cotés, etc.

L'enceinte 101 peut être flexible et peut se déformer facilement. Par exemple, l'enceinte 101 peut être fait avec un matériel flexible. Par exemple, le matériel flexible peut être du plastique, du carton, du métal etc. L'enceinte 101 peut avoir une porte 107. La porte 107 permet d'ouvrir ou de fermer l'enceinte 101. En ouvrant la porte 107, un utilisateur a accès à l'enceinte 101. La porte 107 peut avoir un dispositif de fermeture manuelle ou automatique. L'enceinte 101 a un plafond 111. Le plafond 111a une surface intérieure et une surface extérieure.

L'enceinte 101 a un sol 113. Le sol 113 a une surface intérieure et une surface extérieure. L'enceinte a des murs. Par exemple, l'enceinte 101 a un des murs 106 et 109. Les murs 106 et 109 peuvent avoir chacune une surface intérieure et une surface extérieure. La surface intérieure des murs 106 et 109 peut avoir un dispositif pour recevoir un pilulier. Par exemple, les surfaces intérieures des murs 106 et 109 peuvent avoir des rails pour recevoir un pilulier.

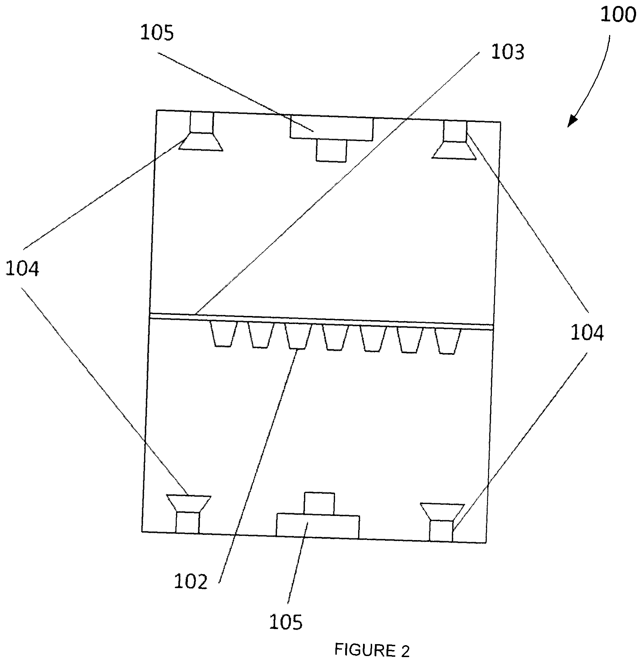

Le système d'identification 100 peut inclure un pilulier 103. Le pilulier 103 peut être intégré dans l'enceinte. Le pilulier 103 peut aussi être une pièce détachée de l'enceinte. Le pilulier 103 a des cases 102. Par exemple, une case 102 peut être intégrée dans le pilulier. La case reçoit au moins un médicament qui est déposé dans le pilulier. Se réfèrent à la Figure 2, la case 102 à une ouverture pour recevoir un ou plusieurs médicaments. La case 102 a aussi un fond qui permet aux médicaments de s'accumuler à l'intérieur de la case 102. La case 102 a aussi des murs. Les murs définissent le volume intérieur de la case 102. Le pilulier peut être transparent. Les cases des piluliers peuvent être transparentes.

Le système d'identification de médicaments inclut un ou plusieurs dispositifs de mesure. Un dispositif de mesure sert à identifier un ou plusieurs médicaments déposés dans un pilulier. Par exemple, un dispositif de mesure peut capter une image d'un médicament déposé dans le pilulier et, par la suite, analyser les caractéristiques d'identification du médicament pour déterminer de quel médicament il s'agit.

Se référant à la Figure 2, des dispositifs de mesure sont insérés dans l'enceinte 101. Un dispositif de mesure est inséré dans la surface intérieure de plafond 111. Un dispositif de mesure est aussi inséré dans la surface du sol 113.

Un dispositif de mesure peut inclure un système d'éclairage et un système d'imagerie. Le système d'éclairage peut inclure une lampe pour projeter de la lumière à l'intérieur de l'enceinte et sur le pilulier. Se référant à la Figure 2, des systèmes d'éclairage 104 se retrouvent dans le plafond 111 et dans le sol 113. Le système d'imagerie peut inclure des caméras pour prendre des photos ou des vidéos d'un pilulier. Se référant à la Figure 2, des systèmes d'imagerie se retrouvent dans le plafond 111 et dans le sol 113.

Dans la Figure 2, le pilulier 103 se retrouve à l'intérieur de l'enceinte 101. Les systèmes d'éclairage illuminent l'intérieur de l'enceinte et le pilulier à partir du plafond et à partir du sol. Par exemple, des systèmes d'éclairage peuvent projeter de la lumière directement sur le pilulier. Les dispositifs d'imagerie 105 captent des images du pilulier à partir du haut, c'est-à-dire du plafond. Les dispositifs d'imageries captent des images du pilulier à partir du bas, c'est-à-dire du sol.

Selon un mode de configuration, le pilulier est transparent. Les cases du pilulier sont aussi transparentes. De ce fait, des médicaments qui sont déposés dans les cases du pilulier peuvent être vus à partir de n'importe quel point de repère à l'intérieur du l'enceinte. Par exemple, les médicaments peuvent être vus à partir du plafond. Le dispositif d'imagerie peut capter une image du pilulier incluant tous les médicaments à partir du plafond. Les médicaments peuvent être aussi vus à partir du sol de l'enceinte du fait que le pilulier et les cases sont transparents. La visibilité des médicaments est améliorée par le système d'éclairage. Le dispositif d'imagerie peut capter une image de pilulier incluant tous les médicaments à partir du sol de l'enceinte.

L'image du pilulier contenant des médicaments est ensuite analysé pour identifier chaque médicament. Un médicament peut être identifié à partir des caractéristiques propre à ce médicament. Par exemple, un médicament peut avoir une forme, une taille, un couleur, une dimension, une texture ou un marquage qui lui est propre.

Par exemple, chaque médicament a ses caractéristiques d'identification qui lui est propre et qui sont différents de celles des autres. Par exemple, un médicament particulier peut avoir une série de lettres et de nombres marqués. Un médicament particulier peut avoir une inscription d'une couleur alternée ou d'une même couleur que celle du médicament lui-même. L'inscription peut être creusée à la surface. Chaque médicament peut avoir une forme particulière. Par exemple, la forme du médicament peut être arrondie, ovale, triangulaire ou une autre forme.

Chaque médicament peut avoir une épaisseur particulière. Le dispositif de mesure peut communiquer l'image du pilulier à un système informatique pour identifier les médicaments dans le pilulier. Le système informatique isole chaque médicament dans l'image. Pour chaque médicament isolé, le système informatique identifie ce médicament à partir des caractéristiques propres à ce médicament.

Par exemple, dans une image de pilulier prise par le haut, le système informatique détermine qu'il y a un médicament de couleur rouge, ayant une forme ovale et une inscription « FM ». A partir de ces caractéristiques, le système informatique peut déterminer que le médicament est un médicament de type Y. Par exemple, dans une image du pilulier prise par le bas, le système informatique détermine que le médicament a une forme de capsule, ayant une couleur rose d'un côté et une couleur blanche d'un autre côté. Le système informatique détermine aussi qu'il y une inscription « P 087 » sur la médication. A partir de ces caractéristiques, le système informatique peut déterminer que le médicament est un diphenhydramique.

Dans une configuration, le système d'identification de médicaments inclut un système de comparaison pour comparer les images du pilulier prises sous différents angles pour mieux identifier les médicaments. Par exemple, les images du pilulier peuvent être prises sous différents angles du haut, du bas, ou latéralement. Par exemple, un pilulier contient des médicaments et un dispositif d'imagerie peut se situer dans le plafond de l'enceinte et prend une première image du pilulier à partir du haut. De même, un autre dispositif d'imagerie peut se situer dans le sol de l'enceinte et prend une deuxième image du pilulier à partir du bas. La première et la deuxième image sont envoyés au système de comparaison pour identifier les médicaments qui sont dans le pilulier.

Le système de comparaison peut inclure un dispositif de comparaison incluant un système informatique. Par exemple, le dispositif de comparaison peut recevoir deux images du pilulier : une image qui a été prise à partir du haut du pilulier; et une image qui a été prise à partir du bas. Du fait que des médicaments peuvent s'empiler les uns sur les autres dans une case du pilulier, la comparaison des deux images permet d'avoir une meilleure perspective du contenu du pilulier ou des cases du pilulier. Par exemple, à partir des images comparés, on peut déterminer le nombre de médicaments dans chaque case du pilulier et identifier chaque médicament séparément.

Par exemple, le système informatique peut utiliser l'image prise à partir du haut du pilulier pour identifier les caractéristiques d'un médicament. A partir de l'image du haut, le système informatique peut déterminer qu'un médicament a une couleur rouge et une forme ovale. De même, le système informatique peut déterminer qu'un médicament a une inscription, par exemple « P087 », sur sa surface. A titre d'exemple, à partir de l'image du bas, le système informatique peut déterminer qu'un médicament a une couleur blanche et une rectangulaire.

Le système informatique peut utiliser l'image prise à partir du bas du pilulier pour valider les conclusions par rapport à l'image prise à partir du haut. Par exemple, en utilisant l'image prise à partir du bas du pilulier, le système informatique valide que le médicament a effectivement une couleur rouge et une forme ovale.

Par exemple, à partir des deux images, le système informatique peut déterminer le nombre de médicaments dans chaque case du pilulier et les caractéristiques de chaque médicaments. Par exemple, à partir des deux images, le système informatique peut déterminer qu'il y a deux médicaments dans la case Al du pilulier, les deux médicaments sont l'un par-dessus l'autre, celui du haut est rouge et a une inscription « P087 » sur sa surface supérieur, et celui du bas est vert et a une forme ovale.

Le système informatique compare les caractéristiques de chaque médicaments avec des signatures de médicaments dans une base de données qui referme des signatures caractéristiques permettant d'identifier un médicament.

Une signature caractéristique d'un médicament est un ensemble de caractéristiques qui sont propres à ce médicament. En déterminant, les caractéristiques d'un médicament, le système informatique peut comparer ces caractéristiques à des signatures caractéristique prédéfinis pour identifier un médicament.

Dans une configuration, le système d'identification de médicaments inclut un système de validation pour comparer le médicament identifié à une prescription. Par exemple, après avoir identifié le contenu d'une case de pilulier, le système de validation vérifie si ces médicaments correspondent à une prescription.

Par exemple, le pilulier ou une case du pilulier pourrait correspondre à une prescription définie. Par exemple, une prescription contient deux médicaments de type Y. Le système inclut un dispositif de validation pour obtenir un contenu identifié pour le contenu de la case et comparer le contenu identifié à une prescription. Par exemple, le système de validation peut vérifier que deux médicaments de type Y sont dans le pilulier ou dans une des cases du pilulier.

Par exemple, une prescription définit qu'un médicament de type Z et un médicament de type P doit être pris deux fois par jours. Le système informatique peut être configuré pour associer des cases du pilulier à chaque période ou les médicaments doivent être prises. Par exemple, le système informatique peut associer les cases A1 et A2 du pilulier à deux périodes de la journée ou les médicaments doivent être prises. Ensuite, le système informatique peut déterminer si les médicaments qui sont dans les cases A1 et A2 correspondent effectivement à un médicament de type Z et un médicament de type P, tel que défini dans la prescription.

### Exemple 2 - Séquences d'opération de la reconnaissance des médicaments dans les cases

### Méthode avec boîtier fermé ou enceinte

Le contenu du pilulier de médicaments est préparé selon la prescription validée par le pharmacien. Le pilulier est disposé dans le support du boîtier fermé. L'acquisition des données est déclenchée par l'opérateur. La séquence d'acquisition peut se faire de façon alternée ou simultanée pour chacune des dimensions et/ou chacune des cases.

Le(s) capteur(s) placé(s) en haut transmet au système les données (voir Figures 1 et 2). Il identifie le ou les caractéristiques des médicaments. Le(s) capteur(s) placé(s) en bas transmet au système les données. Il identifie le ou les caractéristiques des médicaments.

Le système compare et analyse les couples de caractéristiques obtenues précédemment et il assemble l'ensemble des caractéristiques des médicaments présents dans la case du pilulier.

Le système compare les caractéristiques obtenues avec les caractéristiques signatures de la banque de données.

Le système identifie le contenu de la case.

Le système compare le contenu identifié de la case avec la prescription.

Le système valide ou non le contenu de la case.

La séquence est répétée pour chacune des cases du blister alternativement ou simultanément.

### Exemple 3 - Séquence d'opération de la reconnaissance des médicaments dans les cases

### Méthode avec plateau de préparation

Le contenu du grand plateau de préparation est préparé selon la prescription validée par le pharmacien.

Le grand plateau de préparation est disposé sur le support du plateau de préparation.

L'acquisition des données est déclenchée par l'opérateur. La séquence d'acquisition peut se faire de façon alternée ou simultanée pour chacune des cases.

Le(s) capteur(s) placé(s) à l'aplomb du grand plateau transmet au système les données. Il identifie la ou les caractéristiques des médicaments.

Le système compare et analyse les caractéristiques obtenues précédemment et il assemble l'ensemble des caractéristiques médicaments présents dans la case du grand plateau.

Le système compare les caractéristiques obtenues avec les caractéristiques signatures de la banque de données.

Le système identifie le contenu de la case.

Le système compare le contenu identifié de la case avec la prescription.

Le système valide ou non le contenu de la case.

La séquence est répétée pour chacune des cases du blister ou pilulier alternativement ou simultanément.

### Exemple 4 - Méthode avec plateau

### Détail du système

Dans une configuration, le système de la présente divulgation comprend:
- un plateau de préparation vérification présente un nombre de cellules équivalentes au blister carte alvéolée en préparation, généralement 28 cases (7 x 4 cm). La taille du plateau est approximativement de 32 x 35 cm (28 cellules de 8 x 5 cm) pour permettre de contenir l'ensemble des médicaments d'une prescription sans chevauchement;
- un tiroir rétractable sous le plateau pour libérer les médicaments du plateau vers le pilulier.
- un ensemble de tubes assurant la correspondance entre les cellules du plateau de préparation et les cases du pilulier;
- un système de vérification par numérisation optique pour vérifier le contenu du plateau de préparation, le système optique peut être fixe ou mobile et se déplacer sur deux axes horizontaux afin de contrôler toute la surface des cellules du plateau; et une balance de précision peut être ajoutée pour contrôler le poids de l'ensemble des médicaments disposés sur le plateau; et
- un support du pilulier pour assurer la correspondance exacte des tubes descendant du plateau de vérification vers le blister.

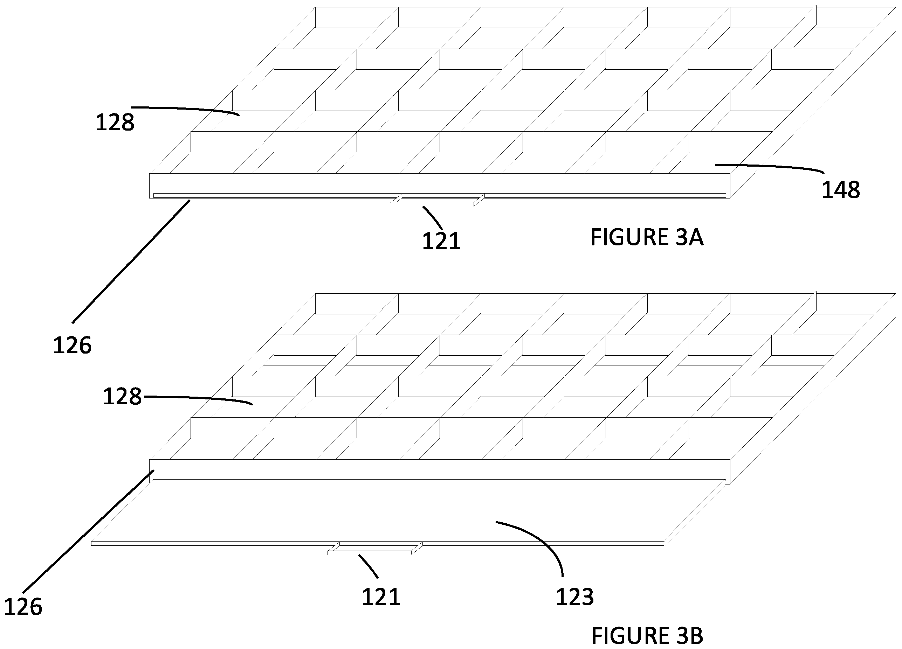

Dans une configuration, le système d'identification de médicaments inclut un compartiment capable de recevoir au moins un médicament. Le compartiment peut être un plateau de préparation ou un compartiment d'un plateau de préparation. Se référant à la Figure 3A, il est divulgué un plateau de préparation 126. Le plateau de préparation inclut un tiroir rétractable. Se référant à la Figure 3B, le tiroir 123 est rétracté. Un support 121 permet de tirer le tiroir 123 pour le rétracter.

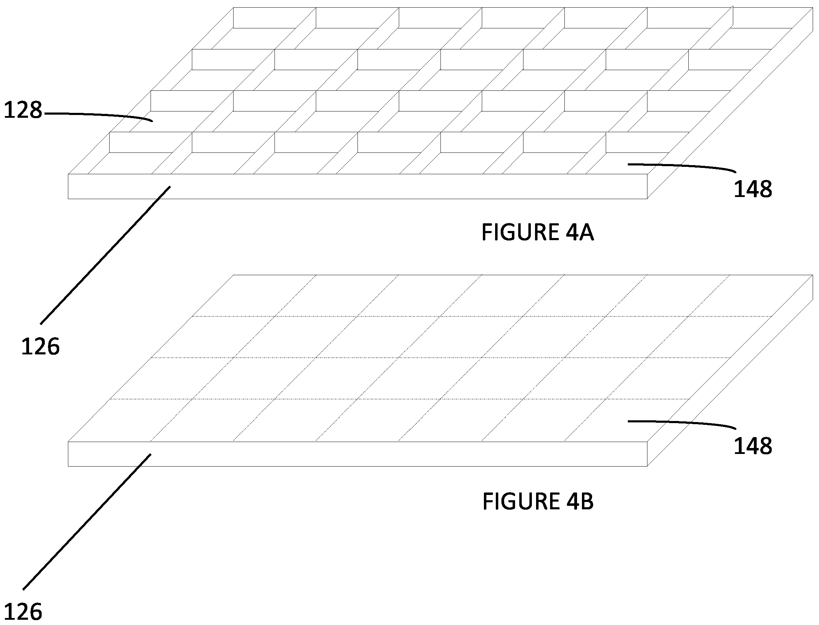

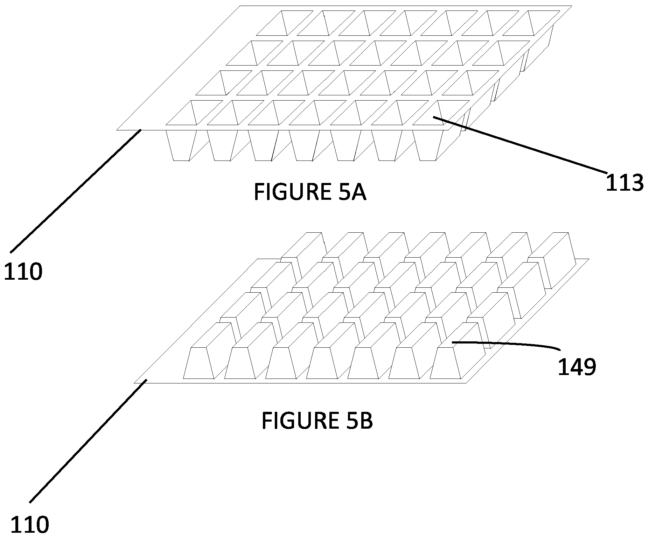

La Figure 4A montre un exemple de plateau de préparation incluant des compartiments 128. La Figure 4B montre un exemple de plateau de préparation dans une position inversée. La Figure 5A montre un exemple de pilulier 110 incluant des cases 113. La Figure 5B montre un exemple de pilulier 110 dans une position inversée.

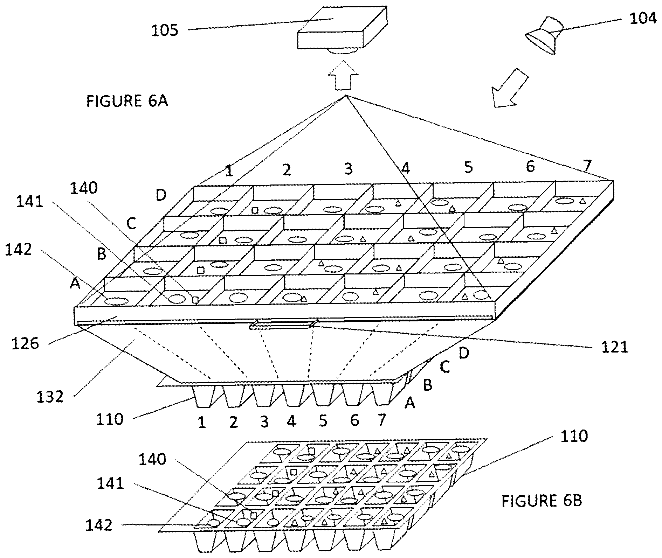

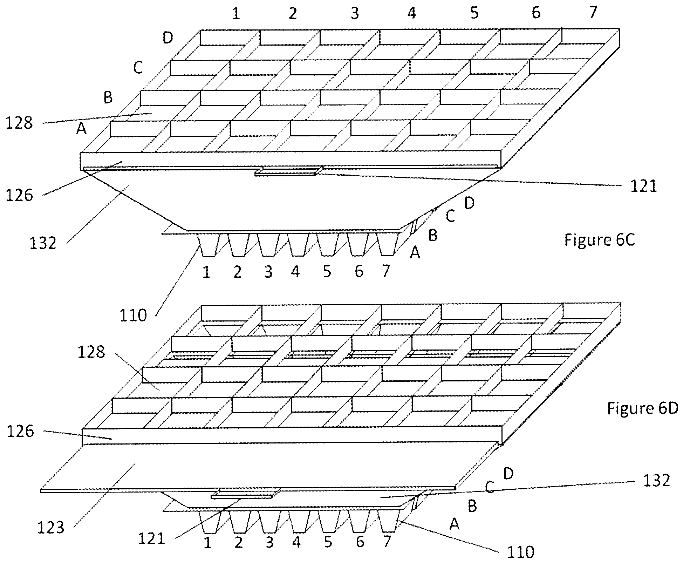

Se référant à la Figure 6A, il est divulgué un système d'identification de médicaments. Des médicaments 140, 141 et 142 sont déposés dans le plateau de préparation 126. Un système d'éclairage 104 projette de la lumière sur le plateau et un système d'imagerie 105 prends des photos ou des vidéos du plateau de préparation126. A partir des images captées par le système d'imagerie, un dispositif d'identification identifie les médicaments sur le plateau de préparation 126 pour analyser leurs caractéristiques d'identification. Par exemple, le dispositif d'identification peut déterminer qu'un médicament a une couleur rouge et une forme ovale et une inscription « P087 ». Un système informatique compare ces caractéristiques avec des signatures de médicaments dans une base données. Ceci permet de valider le contenu du plateau de préparation et d'identifier les médicaments sur le plateau de préparation.

Un dispositif de validation compare les médicaments identifiés à une prescription. Ceci assure que chaque compartiment du plateau de préparation contient les médicaments de la prescription qui lui corresponde. Une prescription peut être associée au plateau de préparation ou à chaque compartiment du plateau de préparation. Le système d'identification de médicaments permet de vérifier que les médicaments correspondant à une prescription particulière se retrouvent dans le compartiment correspondant à cette prescription,

Un dispositif de connexion permet à un médicament situé dans un compartiment de passer par gravité, du compartiment vers une case d'un pilulier. Un tiroir peut être rétracté pour permettre aux médicaments de passer le dispositif de connexion.

Se référant à la Figure 6A et 6D, un tiroir rétractable 123 a un support/poignet 121. En rétractant le tiroir 123 par support/poignet 121, les médicaments passent par gravité, du plateau de préparation 126 vers un pilulier 110. Un dispositif de connexion 132 est un conduit à passages permettant aux médicaments situés sur le plateau de préparation de passer dans les cases du pilulier 110.

Par exemple, se référant à la Figure 6A, le médicament 142 se retrouve à la case Al du plateau de préparation et les médicaments 140 et 141 se retrouvent à la case A2 du plateau de préparation. Le système d'identification de médicaments identifie le médicaments 142 et valide qu'il correspond à la prescription associée à la case Al. De même, le système d'identification de médicaments identifie les médicaments 140 et 141, et valide qu'ils correspondent à la prescription associé à la case A2. Une fois la validation terminée, le tiroir est rétractée pour permettre aux médicaments de passer aux cases correspondantes du pilulier 110. Le tiroir peut être rétracté de façon automatique. Le tiroir peut être rétracté par un utilisateur.

Se référant à la Figure 6B, on voit une vue du haut du pilulier 110 de la Figure 6A après que le tiroir a été rétracté. De ce fait, le médicament 142 se retrouve à la case Al du pilulier et les médicaments 140 et 141 se retrouvent à la case A2 du pilulier.

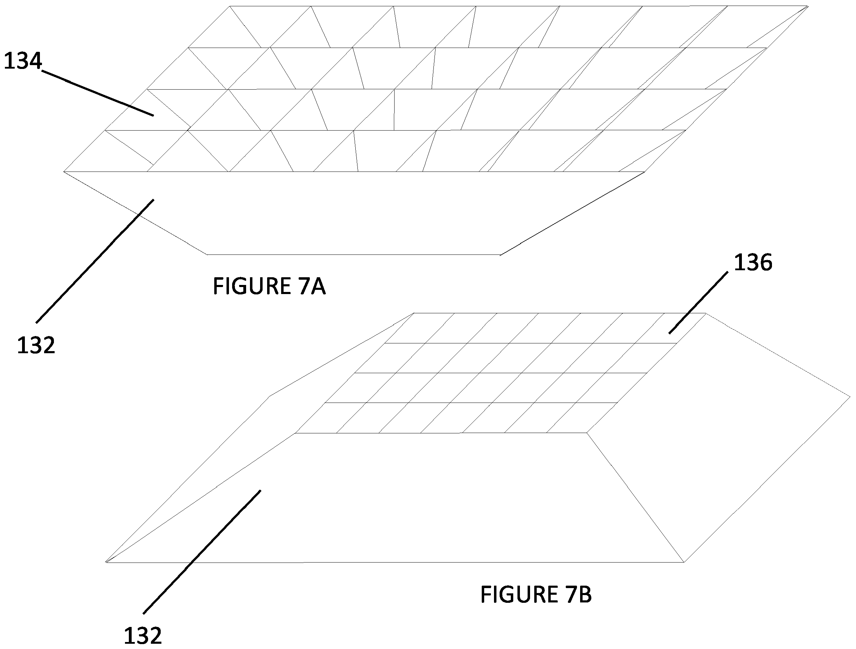

Se référant à la Figure 7A, un dispositif de connexion 132 inclue plusieurs orifice 134. Le dispositif de connexion peut être un réducteur. La Figure 7B montre le dispositif de connexion 132 dans une position renversée. Le dispositif de connexion 132 a un côté supérieure et un côté inférieur. Le côté supérieure plusieurs orifices 134. Le côté inférieur a des orifices 136. Chaque orifice 134 du côté supérieure correspond à un orifice 136 du côté inférieur. Par exemple, il y a des couloirs à l'intérieur du réducteur , ce qui permet à chaque orifice 134 du côté supérieure de communiquer directement à un orifice 136 du côté inférieur. Par exemple, quand un médicament arrive à un orifice 134, le médicament passe par un couloir pour sortir par un orifice 136.

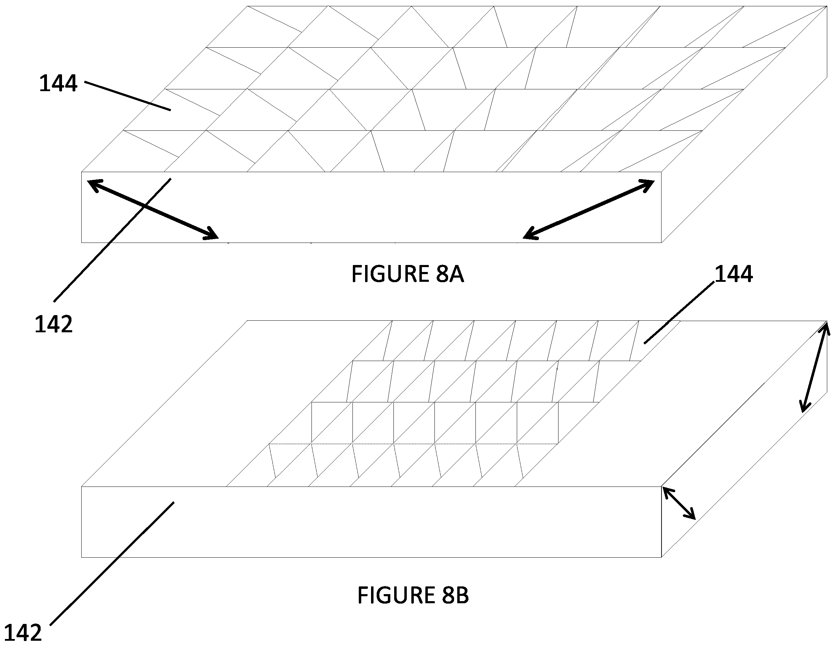

Se référant à la Figure 8A, un dispositif de connexion 142 inclue plusieurs orifice 144. La Figure 8B montre le dispositif de connexion 142 dans une position renversée. Les couloirs des orifices 144 ont une pente plus pointues, ce qui permet de réduire la surface totale du côté inférieur du dispositif de connexion 142.

Le dispositif de connexion est un dispositif de connexion qui peut se connecter au plateau de préparation et à un pilulier. Le côté supérieur du dispositif de connexion peut se connecter au plateau de préparation. Le côté inférieur du dispositif de connexion peut se connecter à un pilulier. Dans une configuration , le réducteur peut se connecter au plateau de préparation et comporte le même nombre d'orifices que de compartiments ou un nombre d'orifices supérieur au nombre de compartiments du plateau de préparation. Le dispositif de connexion peut se connecter à un pilulier et comporte le même nombre d'orifices que de cases ou un nombre d'orifices supérieur au nombre de cases du pilulier.

Se référant à la Figure 7A, les orifices 134 sont dimensionnés pour correspondre aux compartiments 128 du plateau des préparation 126. Par exemple, le dispositif de connexion 132 va se positionner en dessous du plateau de préparation 126. Chaque compartiment 128 du plateau de préparation 126 a une surface, ayant une longueur et une largeur. La surface permet de déposer des médicaments. Quand le dispositif de connexion 132 est positionné en dessous du plateau de préparation 126, la surface de chaque orifice 134 du réducteur correspond à la surface de chaque compartiment 128.

Se référant aux Figures 3A et 4A, la surface 148 du compartiment du plateau de préparation est plus grand que la surface 149 de la case d'un pilulier, par exemple montré dans la Figure 5B. Ainsi, dans une configuration, la surface inférieure 148 du compartiment est supérieure à une surface inférieure de la case 149 du pilulier. De même, le volume interne défini par le compartiment est supérieur au volume interne défini par la case du pilulier.

Le dispositif de connexion permet le passage d'un ou plusieurs médicaments depuis un compartiment d'un deuxième distributeur vers son vis-à-vis d'un premier distributeur. Par exemple, le dispositif de connexion permet le passage d'un ou plusieurs médicaments depuis un plateau de préparation vers un pilulier. Le passage peut se faire par gravité.

Se référant à la Figure 6A, on voit un dispositif de connexion 132 positionné en dessous du plateau de préparation 126. Par exemple, quand le tiroir 121 est rétracté, les médicaments qui sont dans le plateau de préparation vont descendre dans les orifices du dispositif de connexion. Ensuite, les médicaments vont se retrouver dans la case correspondante du pilulier, tel que contre montré dans la Figure 6B.

### Étapes de mise en œuvre de la méthode d'utilisation

1. Un blister distributeur de médicament à remplir est disposé dans le support;
2. En fonction de la prescription, les médicaments sont disposés par le technicien sur le plateau dans les cellules correspondantes du plateau;
3. Les médicaments disposés sur le plateau sont comptés et vérifiés par le système de numérisation optique comme décrit dans le système EyeCon® commercialisé par Avery Weigh-Tronix ;
4. Validation du contenu par le système de vérification par rapport à la prescription;
5. Ouverture du tiroir rétractable par l'opérateur;
6. Les médicaments disposés sur le plateau tombent par gravité dans les cases correspondantes du blister carte alvéolée en suivant le chemin des tubes; et
7. Le blister distributeur de médicament est refermé par une feuille d'étanchéité par l'opérateur puis retiré du support.

Un système d'identification et de validation de médicaments disposés, selon une prescription, dans les compartiments d'un distributeur comprenant n compartiments, n étant un nombre entier supérieur ou égal à 1, chacun des médicaments étant susceptible d'être au moins partiellement superposé avec au moins un autre médicament présent dans le même compartiment du distributeur, ledit système inclut:
- un dispositif de mesure configuré pour recueillir des caractéristiques d'identification des médicaments disposés dans chacun des compartiments du distributeur;
- un dispositif de transmission configuré pour transférer, à une unité de calcul, les caractéristiques d'identification recueillies par le dispositif de mesure;
- une unité de calcul configurée pour déterminer, à partir des caractéristiques d'identification recueillies si chacun des médicaments effectivement présents dans chacun des compartiments correspond ou non à un médicament qui, selon la prescription, devrait s'y trouver et/ou pour indiquer dans quel compartiment se trouve la ou les non-correspondances et/ou quelle est la nature de la ou des non-correspondances.

Le système d'identification et de validation peut comprendre:
- un dispositif de mesure configuré pour recueillir, de façon isolée ou par paquets, et éventuellement pour analyser, les caractéristiques d'identification des médicaments disposés dans chacun des compartiments du distributeur:
- qu'un des médicaments présents dans un compartiment soit séparé ou non, par un espace libre, d'un ou plusieurs médicaments voisins et/ou
- qu'un des médicaments accote et/ou chevauche partiellement ou totalement avec un ou plusieurs médicament(s) adjacent(s);
- un dispositif de transmission configuré pour transférer, à une unité de calcul, les caractéristiques d'identification recueillies par le dispositif de mesure, analysées ou non analysées ou partiellement analysées;
- une unité de calcul_configurée pour:
- isoler et/ou analyser les caractéristiques d'identification reçues du dispositif de transmission pour chacun des médicaments effectivement présents dans un compartiment du distributeur, et identifier chacun de ces médicaments,
   i. - comparer les caractéristiques isolées et/ou analysées dans l'étape précédente avec les informations contenues dans une base de données, interne à l'unité de calcul ou externe, des caractéristiques des médicaments devant, selon la prescription, être présents dans un des compartiments du distributeur.

Le système d'identification et de validation étant configuré pour déterminer si:
- tous les médicaments effectivement présents dans chacun des compartiments correspondent ou non à ceux qui, selon la prescription, devraient s'y trouver; et/ou
- le nombre de médicaments effectivement présents dans un compartiment correspond au nombre de médicament qui devrait, selon la prescription, s'y trouver; et/ou
- le nombre total de médicaments présents dans tous les compartiments du distributeur correspond au nombre total de médicaments qui devraient, selon la prescription, se trouver dans le distributeur; et/ou
- le poids des médicaments présents dans un compartiment correspond au poids des médicaments qui devraient, selon la prescription, s'y trouver et/ou
- le poids de tous les médicaments présents dans tous les compartiments du distributeur correspond au poids total de tous les médicaments, qui selon la prescription, devraient s'y trouver.

Les caractéristiques d'identification des médicaments sont : des caractéristiques géométriques telles que des formes cachées, des formes partiellement cachées ou des formes complètes, la taille, la couleur, les dimensions, ou bien la texture, ou bien un ou plusieurs marquages et la luminescence. Le dispositif de mesure comprend au moins un capteur et, de préférence, comprend plusieurs capteurs positionnés dans un même plan ou dans des plans décalés. Les capteurs sont, par rapport au plan dans lequel est positionné le distributeur, positionnés: au-dessus; au-dessous; et/ou llatéralement. Au moins un des capteurs peut être déplacé dans différentes positions de l'espace. Le distributeur à une structure au moins partiellement souple et/ou pliable permettant de déformer au moins partiellement le distributeur, et au besoin

Le dispositif de mesure est au moins un dispositif d'analyses chimiques et/ou physiques choisi, de préférence, dans le groupe constitué par les moyens suivants: ,
- un capteur 2D lumière visible (photo ou vidéo),;
- un capteur 3D lumière visible
- un appareil Rayon X, tel que décrit notamment dans un des documents suivants dont tout le contenu est incorporé par référence à la présente demande;
- un appareil de décomposition RGB, tel que décrit notamment dans un des documents suivants dont tout le contenu est incorporé par référence à la présente demande;
- - un analyseur des couleurs avec illumination par des lumières de différentes couleurs, incluant la lumière blanche, lumière ultraviolette, lumière rouge, lumière verte, lumière bleue et/ou lumière infrarouge, lumière stroboscopique;
- un appareil de reconnaissance optique des caractères (OCR) et /ou de vérification optique des caractères (OCV), tel que décrit notamment dans un des documents suivants dont tout le contenu est incorporé par référence à la présente demande
- un spectroscope (infrarouge proche ou fluorescence) tel que décrit notamment dans un des documents suivants dont tout le contenu est incorporé par référence à la présente demande:;
- un spectroscope MMS (multimodal multiplex sampling) tel que décrit notamment dans un des documents suivants dont tout le contenu est incorporé par référence à la présente demande ;
- un appareil d'Imagerie par résonnance magnétique;
- un appareil à ultrasons;
- un scanner par excitation laser; et
- un appareil de pesée de précision.

Le premier et le deuxième distributeur comprennent le même nombre de compartiments. Le premier et le deuxième distributeur comprennent chacun un nombre de compartiments qui est avantageusement compris entre 7 et 28. Le dispositif de mesure est un dispositif de numérisation optique comme décrit dans le système EyeCon commercialisé par la société Avery Weight-Tronix. Le système d'identification et de validation de médicaments est utilisé pour réduire le risque d'erreur lors de remplissage d'un distributeur de médicament selon une prescription.

Une méthode de validation de la conformité, à une prescription médicale, du remplissage d'un distributeur de médicament, la méthode inclue les étapes suivantes:
- de remplissage, selon une prescription médicale, des compartiments d'un distributeur, par un opérateur et/ou par un automate;
- de détermination, grâce à la mise en œuvre d'au moins un des systèmes d'identification du contenu de chacun des compartiments; et
- d'envoi d'un message d'erreur et/ou de correction éventuelle du contenu d'un ou de plusieurs compartiment(s) du distributeur en cas de non-conformité constatée par ledit système par rapport à la prescription médicale.

La détermination du contenu de chacun des compartiments se fait grâce à la mise en œuvre d'un système d'identification et les mesures correspondantes sont réalisées par secteur du récipient, chaque secteur pouvant comprendre un ou plusieurs compartiments.

Une méthode de validation de la conformité à une prescription, dans laquelle la détermination du contenu de chacun des compartiments se fait grâce à la mise en œuvre du système d'identification et les mesures correspondantes sont réalisées après pliage, au moins partiel, d'un secteur du contenant, le pliage se faisant avantageusement selon la ligne de séparation de rangées de compartiments, la ligne correspondante de séparation étant, de préférence, réalisée à partir d'un matériau souple et/ou à partir d'un matériau susceptible de s'assouplir, notamment par chauffage thermique.

Un système à étages d'identification et de validation de médicaments disposés, selon une prescription, dans les compartiments d'un distributeur destiné à un patient et/ou destiné à un accompagnateur du patient, lesdits médicaments étant susceptibles d'être au moins partiellement superposés et/ou accotés avec au moins un autre médicament présent dans le même compartiment du distributeur, le système comprend:
- un premier distributeur destiné au patient et/ou à un accompagnateur du patient ;
- un deuxième distributeur surdimensionné (par rapport à la taille du premier distributeur) comportant le même nombre de compartiments ou un nombre de compartiments supérieur au nombre de compartiments du premier distributeur, ledit deuxième distributeur étant, de préférence d'une taille suffisante pour éviter le recouvrement de médicaments, et de préférence, positionné à une hauteur plus grande que le premier distributeur, et avantageusement, au-dessus et/ou décalé;
- un dispositif de connexion entre les compartiments du premier distributeur et ceux du deuxième distributeur, ledit dispositif de connexion permettant le transit, de préférence par gravité, d'un ou plusieurs médicaments depuis un compartiment du deuxième distributeur vers son vis-à-vis du premier distributeur, ledit dispositif de connexion étant de préférence constitué par un faisceau de conduits qui sont de préférence sensiblement parallèles, chacun des conduits étant:
   - au niveau de sa partie supérieure solidarisée avec la partie inférieure d'un des compartiments du deuxième distributeur, et
   - au niveau de sa partie inférieure solidarisée avec la partie supérieure du compartiment du premier distributeur en vis-à-vis;
La partie inférieure d'un compartiment du deuxième distributeur surdimensionné est configurée de façon à disposer d'un dispositif libérateur laissant, à la demande, les médicaments d'un des compartiments du deuxième distributeur migrer vers le compartiment en vis -à-vis du premier distributeur, et l'ouverture supérieure d'un conduit du dispositif de connexion est, de préférence, plus grande que l'orifice inférieur du conduit de connexion;
- un dispositif de mesure configuré pour analyser les caractéristiques d'identification des médicaments disposés de façon à ne pas se chevaucher dans chacun des compartiments du distributeur surdimensionné : qu'un des médicaments présents dans un compartiment soit séparé par un espace d'un ou plusieurs médicaments voisins ou qu'il soit au contact avec un ou plusieurs médicament(s) adjacent(s) ;
- un dispositif de transmission configuré pour transférer les caractéristiques d'identification (analysées ou non analysées) recueillies par le dispositif de mesure et relatives aux médicaments, présents dans les compartiments du deuxième distributeur, à une unité de calcul;
- une unité de calcul_configurée pour:
   - isoler les caractéristiques d'identification reçues du dispositif de transmission pour chacun des médicaments effectivement présents dans un compartiment du deuxième distributeur, et d'identifier chacun de ces médicaments,
   - comparer les caractéristiques isolées dans l'étape précédente avec les informations contenues dans une base de données des caractéristiques des médicaments devant, selon la prescription, être présents dans un des compartiments du distributeur, et
   - ledit système d'identification et de validation émettant un message selon lequel :
- tous les médicaments effectivement présents dans chacun des compartiments du deuxième distributeur correspondent ou non à ceux, qui selon la prescription, devraient s'y trouver;
   i. le nombre de médicaments effectivement présents dans un compartiment correspond au nombre de médicaments qui devraient, selon la prescription, s'y trouver; et/ou
- le nombre total de médicaments présents dans tous les compartiments du distributeur correspond au nombre total de médicaments qui devraient, selon la prescription, se trouver dans le distributeur; et/ou
- le poids des médicaments présents dans un compartiment correspond au poids des médicaments qui devrait, selon la prescription, s'y trouver et/ou
- le poids de tous les médicaments présents dans tous les compartiments du distributeur correspond au poids total de tous les médicaments, qui selon la prescription, devraient s'y trouver.

Un procédé de fabrication du système à étages d'identification et de validation par mise en œuvre d'au moins une des techniques connues d'assemblage tels que notamment: le vissage, le collage, le boulonnage, l'emboîtage, le rivetage et la soudure.

Le système à étage d'identification et de validation de médicaments est utilisé pour réduire le risque d'erreur lors de remplissage d'un distributeur de médicaments selon une prescription.

Une méthode de validation de la conformité à une prescription médicale, du remplissage d'un distributeur de médicament, ladite méthode comprend les étapes suivantes:
- de remplissage selon une prescription médicale des compartiments d'un distributeur surdimensionné;
- de détermination du contenu de chacun des compartiments du système surdimensionné grâce à la mise en œuvre d'un des systèmes d'identification et/ou de mesure selon la présente divulgation; et/ou
- d'envoi d'un message de non de non-conformité constatée par rapport à la prescription médicale; et/ou
- de correction éventuelle du contenu d'un des compartiments du distributeur en cas de non-conformité constatée par rapport à la prescription médicale; et
- de transit des médicaments présents dans un compartiment du distributeur surdimensionné dans le compartiment vis-à-vis d'un distributeur de taille standard.

La détermination du contenu de chacun des compartiments se fait grâce à la mise en œuvre d'un des systèmes à étages d'identification tel que défini dans la présente divulgation, et les mesures correspondantes sont réalisées par secteur du récipient.

La détermination du contenu de chacun des compartiments se fait grâce à la mise en œuvre d'un des systèmes à étages d'identification et les mesures correspondantes sont réalisées avec pliage au moins partiel d'un secteur du contenant.

Un équipement d'aide au contrôle de la conformité du contenu d'un distributeur de médicaments à une ordonnance, comporte:
- un premier et un deuxième distributeur de médicaments, le deuxième distributeur étant surdimensionné par rapport au premier distributeur;
- - un dispositif de connexion entre les compartiments du premier distributeur et ceux du deuxième distributeur, ledit dispositif permettant le transit, de préférence par gravité, d'un ou plusieurs médicaments depuis un compartiment du deuxième distributeur surdimensionné vers son vis à vis du premier distributeur, ledit dispositif de connexion étant de préférence constitué par un faisceau de conduits chacun des conduits du faisceau étant:
- au niveau de sa partie supérieure solidarisé avec la partie inférieure d'un des compartiments du deuxième distributeur surdimensionné, et
   i. - au niveau de sa partie inférieure solidarisé avec la partie supérieure d'un compartiment du premier distributeur en vis-à-vis,
la partie inférieure d'un compartiment du distributeur surdimensionné étant configuré de façon a disposé d'un dispositif libérateur laissant, à la demande, les médicaments d'au moins un des compartiments du deuxième distributeur migrer vers le compartiment en vis-à-vis du premier distributeur, et
l'ouverture supérieure d'un conduit de connexion étant de préférence plus grand que l'orifice inférieur du conduit de connexion.

L'équipement d'aide au contrôle de la conformité est une plaque coulissante du plancher des compartiments du distributeur surdimensionné et la plaque coulissante pouvant se déplacer horizontalement dans des rainures parallèles situées dans le prolongement des murs latéraux de 2 . Les conduits du faisceau de conduits sont faits d'un matériau souple et/ou déformable qui est de préférence un matériau au moins partiellement transparent. La taille du deuxième distributeur surdimensionné est 1,5 à 3,5 fois la taille du premier distributeur. La distance entre la partie inférieure du deuxième distributeur surdimensionné et la partie supérieure du premier distributeur est comprise entre 10 et 60 cm.

Un procédé de fabrication d'un équipement d'aide au contrôle par mise en œuvre d'au moins une des techniques connues d'assemblage telles que notamment: le vissage, le collage, le boulonnage, l'emboîtage, le rivetage et la soudure.

L'utilisation de l'équipement d'aide au contrôle pour réduire le risque d'erreur lors de remplissage d'un distributeur de médicaments selon une prescription.

Une méthode de validation de la conformité à une prescription médicale, du remplissage d'un distributeur de médicament intégré dans un équipement d'aide au contrôle de la conformité, ladite méthode comprend les étapes suivantes:
- de remplissage des compartiments d'un distributeur surdimensionné selon une prescription médicale;
- de détermination du contenu de chacun des compartiments du distributeur surdimensionné;
- - de correction éventuelle du contenu d'un des compartiments du distributeur surdimensionné en cas de non-conformité constatée par rapport à la prescription médicale; et
- de transit des médicaments présents dans un compartiment du distributeur surdimensionné dans le compartiment du premier distributeur en vis a vis.

Résumé des avantages offerts par les systèmes d'identification et de validation de médicaments selon la présente divulgation, notamment les possibilités de:
- vérification de l'ensemble des compartiments du distributeur de médicaments;
- vérification des médicaments présentant un chevauchement dans le ou les compartiments;
- intégration dans les procédures de travail des pharmacies pour la délivrance au patient des prescriptions; et
- de complémentarité des méthodes de travail des pharmaciens pour réaliser le contrôle et la vérification des distributeurs de médicaments réalisés par les assistants techniques.

Résumé des avantages des systèmes à étages d'identification et de validation de médicaments selon la présente divulgation, notamment les possibilités de:
- vérification de l'ensemble des médicaments qui seront disposés dans les compartiments du distributeur de médicaments;
- aucune possibilité de chevauchement des médicaments présents dans un même compartiment ce qui limite les besoins en calcul et en matériel pour assurer un contrôle et une vérification des médicaments sans faille;
- intégration dans les procédures de travail des pharmacies pour la délivrance au patient des prescriptions et complémentaires des méthodes de travail des pharmaciens pour réaliser le contrôle et la vérification des distributeurs de médicaments réalisés par les assistants techniques; et
- -en cas de coupure de courant et d'opération manuelle, le contrôle et la vérification des médicaments reste possible et est facilité par la disposition du plateau surdimensionné.

La présente divulgation n'est pas limitée à une identification des médicaments par une reconnaissance optique seule, mais peut inclure d'autre méthode ou appareil pour compléter cette reconnaissance comme un laser, un spectromètre.

Bien que la présente divulgation ait été décrite à l'aide de mises en œuvre spécifiques, il est entendu que plusieurs variations et modifications peuvent se greffer aux dites mises en œuvre, et la présente divulgation vise à couvrir de telles modifications, usages ou adaptations de la présente divulgation suivant en général, les principes de la divulgation et incluant toute variation de la présente description qui deviendra connue ou conventionnelle dans le champ d'activité dans lequel se retrouve la présente divulgation, et qui peut s'appliquer aux éléments essentiels mentionnés ci-haut, en accord avec la portée des revendications suivantes.

## Revendications

1. **Système à étages d'identification et de validation de médicaments** disposés, selon une prescription, dans les compartiments d'un distributeur destiné à un patient et/ou destiné à un accompagnateur du patient, ledit système comprenant :
- un premier distributeur (110) destiné au patient et/ou à un accompagnateur du patient;
- un deuxième distributeur (126) surdimensionné par rapport à la taille du premier distributeur, comportant le même nombre de compartiments ou un nombre de compartiments supérieur au nombre de compartiments du premier distributeur;
- un dispositif de connexion (132) entre les compartiments du premier distributeur et ceux du deuxième distributeur, ledit dispositif de connexion permettant le transit d'un ou plusieurs médicaments, depuis un compartiment du deuxième distributeur vers son vis-à-vis du premier distributeur, la partie inférieure d'un compartiment du deuxième distributeur étant configurée de façon à disposer d'un dispositif libérateur laissant, à la demande, les médicaments d'un des compartiments du deuxième distributeur migrer vers le compartiment en vis-à-vis du premier distributeur;
- un dispositif de mesure (105) configuré pour recueillir et analyser des caractéristiques d'identification des médicaments, les médicaments étant disposés de façon à ne pas se chevaucher, dans chacun des compartiments du deuxième distributeur: qu'un des médicaments présents dans un compartiment soit séparé par un espace d'un ou plusieurs médicaments voisins ou qu'il soit au contact avec un ou plusieurs médicament(s) adjacent(s);
- un dispositif de transmission configuré pour transférer les caractéristiques d'identification analysées ou non analysées recueillies par le dispositif de mesure et relatives aux médicaments, présents dans les compartiments du deuxième distributeur, à une unité de calcul; et
- l'unité de calcul configurée pour:
- isoler les caractéristiques d'identification reçues du dispositif de transmission pour chacun des médicaments effectivement présents dans un compartiment du deuxième distributeur, et d'identifier chacun de ces médicaments,
- comparer les caractéristiques d'identification isolées dans l'étape précédente avec les informations contenues dans une base de données des caractéristiques des médicaments devant, selon la prescription, être présents dans un des compartiments du deuxième distributeur, et
ledit système d'identification et de validation étant configuré pour émettre un message selon lequel :
- tous les médicaments effectivement présents dans chacun des compartiments du deuxième distributeur correspondent ou non à ceux, qui selon la prescription, devraient s'y trouver;
- le nombre de médicaments effectivement présents dans un compartiment correspond au nombre de médicaments qui devraient, selon la prescription, s'y trouver;
- le nombre total de médicaments présents dans tous les compartiments du deuxième distributeur correspond au nombre total de médicaments qui devraient, selon la prescription, se trouver dans le deuxième distributeur;
- le poids des médicaments présents dans un compartiment correspond au poids des médicaments qui devrait, selon la prescription, s'y trouver; et/ou
- le poids de tous les médicaments présents dans tous les compartiments du deuxième distributeur correspond au poids total de tous les médicaments, qui selon la prescription, devraient s'y trouver.

2. Système, selon la revendication 1, ledit système ayant au moins une des caractéristiques suivantes :
- les médicaments sont au moins partiellement superposés et/ou accotés avec au moins un autre médicament présent dans le même compartiment du deuxième distributeur;
- ledit deuxième distributeur étant d'une taille suffisante pour éviter le recouvrement de médicaments;
- ledit deuxième distributeur étant positionné à une hauteur plus grande que le premier distributeur;
- ledit deuxième distributeur étant positionné et avantageusement, au-dessus et/ou décalé;
- ledit dispositif de connexion permettant le transit par gravité d'un ou plusieurs médicaments depuis un compartiment du deuxième distributeur vers son vis-à-vis du premier distributeur;
- le dispositif de connexion étant constitué par un faisceau de conduits qui sont de préférence sensiblement parallèles; et
- l'ouverture supérieure d'un conduit du dispositif de connexion étant plus grande que l'orifice inférieur du conduit de connexion.

3. Système, selon la revendication 1 ou 2, dans lequel le premier et le deuxième distributeur comprennent le même nombre pair de compartiments.

4. Système, selon la revendication 3, dans lequel le premier et le deuxième distributeur comprennent un nombre pair de compartiments qui varie de 7 à 28.

5. Procédé de fabrication du système, selon l'une quelconque des revendications 1 à 4, par mise en œuvre d'au moins une des techniques connues d'assemblage telles que notamment: le vissage, le collage, le boulonnage, l'emboîtage, le rivetage et la soudure.

6. Méthode de validation de la conformité à une prescription médicale, du remplissage d'un distributeur de médicament intégré dans un équipement d'aide au contrôle de la conformité, ladite méthode comprenant les étapes suivantes :
- remplir un ou plusieurs médicaments dans des compartiments d'un deuxième distributeur, selon une prescription médicale;
- déterminer le contenu de chacun des compartiments du deuxième distributeur par le biais d'un dispositif de mesure configuré pour analyser des caractéristiques d'identification des médicaments, les médicaments étant disposés de façon à ne pas se chevaucher dans chacun des compartiments du deuxième distributeur: qu'un des médicaments présents dans un compartiment soit séparé par un espace d'un ou plusieurs médicaments voisins ou qu'il soit au contact avec un ou plusieurs médicament(s) adjacent(s);
- transmettre, par le biais d'un dispositif de transmission, les caractéristiques d'identification analysées ou non analysées recueillies par le dispositif de mesure et relatives aux médicaments, présents dans les compartiments du deuxième distributeur, à une unité de calcul, l'unité de calcul étant configurée pour :
- isoler les caractéristiques d'identification reçues pour chacun des médicaments présents dans un compartiment du deuxième distributeur, et d'identifier chacun de ces médicaments,
- comparer les caractéristiques d'identification isolées avec des informations contenues dans une base de données des caractéristiques des médicaments devant, selon la prescription, être présents dans un des compartiments du deuxième distributeur,
- émettre un message selon lequel :
- tous les médicaments effectivement présents dans chacun des compartiments du deuxième distributeur correspondent ou non à ceux, qui selon la prescription, devraient s'y trouver;
- le nombre de médicaments effectivement présents dans un compartiment correspond au nombre de médicaments qui devraient, selon la prescription, s'y trouver;
- le nombre total de médicaments présents dans tous les compartiments du deuxième distributeur correspond au nombre total de médicaments qui devraient, selon la prescription, se trouver dans le deuxième distributeur;
- le poids des médicaments présents dans un compartiment correspond au poids des médicaments qui devrait, selon la prescription, s'y trouver; et/ou
- le poids de tous les médicaments présents dans tous les compartiments du distributeur correspond au poids total de tous les médicaments, qui selon la prescription, devraient s'y trouver
- corriger éventuellement le contenu d'un des compartiments du deuxième distributeur en cas de non-conformité constatée par rapport à la prescription médicale; et
- transiter des médicaments présents dans le compartiment du deuxième distributeur dans le compartiment d'une premier distributeur en vis à vis, le deuxième distributeur étant surdimensionné par rapport au premier distributeur, par le biais d'un dispositif de connexion entre les compartiments du premier distributeur et ceux du deuxième distributeur.

7. Méthode, selon la revendication 6, dans lequel le médicament est de type blister carte alvéolée, lesdits premier et deuxième distributeurs comprenant chacun n compartiments, n étant un nombre entier supérieur ou égal à 7, et ladite méthode comprenant l'étape
- d'envoyer d'un message de conformité ou de non-conformité constatée par rapport à la prescription médicale.

8. Méthode, selon la revendication 7, dans laquelle la détermination du contenu de chacun des compartiments se fait par secteur du récipient.

9. Méthode, selon la revendication 7 ou 8, dans laquelle la détermination du contenu de chacun des compartiments se fait grâce à la mise en œuvre d'un des systèmes à étages d'identification et de validation définis dans l'une quelconque des revendications 1 à 4, et les mesures correspondantes sont réalisées avec pliage au moins partiel d'un secteur du contenant.

10. Système, selon l'une quelconque des revendications 1 à 4, dans lequel le dispositif libérateur est une plaque coulissante constitutive du plancher des compartiments du deuxième distributeur, la plaque coulissante pouvant se déplacer horizontalement dans des rainures parallèles situées dans le prolongement des murs latéraux de 2.

11. Système, selon l'une quelconque des revendications 2 à 4 et 10, dans lequel les conduits du faisceau de conduits sont faits d'un matériau souple et/ou déformable qui est de préférence un matériau au moins partiellement transparent.

12. Système, selon l'une quelconque des revendications 1 à 4, 10 et 11, dans lequel la taille du deuxième distributeur est 1,5 à 3,5 fois la taille du premier distributeur.

13. Système, selon l'une quelconque des revendications 1 à 4 et 10 à 12, dans lequel la distance entre la partie inférieure du deuxième distributeur et la partie supérieure du premier distributeur est comprise entre 10 et 60 cm.

14. Utilisation du système, tel que défini dans l'une quelconque des revendications 1 à 4 et 10 à 13, pour réduire le risque d'erreur lors du remplissage d'un distributeur de médicaments selon une prescription.

## Patentansprüche

1. Etagensystem zur Identifizierung und Validierung von Medikamenten, die gemäß einer Verschreibung in den Fächern eines Verteilers angeordnet sind, der für einen Patienten und/oder einen Begleiter des Patienten bestimmt ist, wobei das System umfasst:
- einen ersten Verteiler (110), der für den Patienten und/oder einen Begleiter des Patienten bestimmt ist;
- einen zweiten Verteiler (126), der in Bezug auf die Größe des ersten Verteilers überdimensioniert ist, aufweisend dieselbe Anzahl an Fächern oder eine Anzahl an Fächern über der Anzahl an Fächern des ersten Verteilers;
- eine Verbindungsvorrichtung (132) zwischen den Fächern des ersten Verteilers und denen des zweiten Verteilers, wobei die Verbindungsvorrichtung den Transit von einem oder mehreren Medikamenten von einem Fach des zweiten Verteilers zu seinem Gegenüber des ersten Verteilers gestattet, wobei der untere Teil eines Fachs des zweiten Verteilers derart ausgelegt ist, dass er über eine Freigabevorrichtung verfügt, die auf Anforderung die Medikamente aus einem der Fächer des zweiten Verteilers in das Fach gegenüber des ersten Verteilers migrieren lässt;
- eine Messvorrichtung (105), die ausgelegt ist, um die Identifikationsmerkmale der Medikamente zu ermitteln und zu analysieren, wobei die Medikamente derart angeordnet sind, dass sie sich in jedem der Fächer des zweiten Verteilers nicht verdecken: dass eins der Medikamente, das in einem Fach vorhanden ist, durch einen Raum von einem oder mehreren benachbarten Medikamenten getrennt ist oder dass es mit einem oder mehreren benachbarten Medikament(en) in Kontakt ist;
- eine Übertragungsvorrichtung, die ausgelegt ist, um die analysierten oder nicht analysierten Identifikationsmerkmale, die von der Messvorrichtung ermittelt wurden und sich auf die Medikamente beziehen, die in den Fächern des zweiten Verteilers vorhanden sind, an eine Recheneinheit zu übertragen; und
- die Recheneinheit, die ausgelegt ist, um:
- die von der Übertragungsvorrichtung erhaltenen Identifikationsmerkmale für jedes der Medikamente, die tatsächlich in einem Fach des zweiten Verteilers vorhanden sind, zu isolieren und jedes dieser Medikamente zu identifizieren,
- die in vorangehendem Schritt isolierten Identifikationsmerkmale mit den Informationen zu vergleichen, die in einer Datenbank der Merkmale der Medikamente enthalten sind, die gemäß der Verschreibung in einem der Fächer des zweiten Verteilers vorhanden sein müssen, und
das Identifizierungs- und Validierungssystem ausgelegt ist, um eine Nachricht zu senden, gemäß der:
- alle Medikamente, die tatsächlich in jedem der Fächer des zweiten Verteilers vorhanden sind, denen entsprechen oder nicht, die sich gemäß der Verschreibung dort befinden müssten;
- die Anzahl an Medikamenten, die tatsächlich in einem Fach vorhanden sind, der Anzahl an Medikamenten entspricht, die sich gemäß der Verschreibung dort befinden müssten;
- die Anzahl insgesamt an Medikamenten, die in allen Fächern des zweiten Verteilers vorhanden sind, der Anzahl insgesamt an Medikamenten entspricht, die sich gemäß der Verschreibung im zweiten Verteiler befinden müssten;
- das Gewicht der Medikamente, die in einem Fach vorhanden sind, dem Gewicht der Medikamente entspricht, die sich gemäß der Verschreibung dort befinden müssten; und/oder
- das Gewicht aller Medikamente, die in allen Fächern des Verteilers vorhanden sind, dem Gewicht insgesamt aller Medikamente entspricht, die sich gemäß der Verschreibung dort befinden müssten.

2. System nach Anspruch 1, wobei das System mindestens eins der folgenden Merkmale hat:
- die Medikamente sind mindestens zum Teil übereinanderliegend und/oder nebeneinanderliegend mit mindestens einem anderen Medikament, das in demselben Fach des zweiten Verteilers vorhanden ist;
- wobei der zweite Verteiler eine Größe hat, die ausreichend ist, um zu verhindern, dass sich die Medikamente überlappen;
- wobei der zweite Verteiler in einer größeren Höhe als der erste Verteiler positioniert ist;
- wobei der zweite Verteiler in vorteilhafter Weise darüber und/oder versetzt positioniert ist;
- wobei die Verbindungsvorrichtung den Transit durch Schwerkraft von einem oder mehreren Medikamenten von einem Fach des zweiten Verteilers zu seinem Gegenüber des ersten Verteilers gestattet;
- wobei die Verbindungsvorrichtung aus einem Bündel an Leitungen besteht, die vorzugsweise etwa parallel sind; und
- wobei die obere Öffnung einer Leitung der Verbindungsvorrichtung größer als das untere Loch der Verbindungsleitung ist.

3. System nach Anspruch 1 oder 2, wobei der erste und der zweite Verteiler dieselbe paarige Anzahl an Fächern umfassen.

4. System nach Anspruch 3, wobei der erste und der zweite Verteiler eine paarige Anzahl an Fächern umfassen, die von 7 bis 28 schwankt.

5. Verfahren zur Herstellung des Systems nach einem der Ansprüche 1 bis 4 durch Durchführung von mindestens einer der bekannten Verbindungstechniken wie insbesondere: Schrauben, Kleben, Verbolzen, Rasten, Nieten und Schweißen.

6. Methode zur Validierung der Konformität mit einer medizinischen Verschreibung des Befüllens eines in eine Konformitätskontrollhilfsausrüstung integrierten Medikamentenverteilers, wobei die Methode die folgenden Schritte umfasst:
- Füllen von einem oder mehreren Medikamenten in Fächer eines zweiten Verteilers gemäß einer medizinischen Verschreibung;
- Bestimmen des Inhalts jedes der Fächer des zweiten Verteilers anhand einer Messvorrichtung, die ausgelegt ist, um Identifikationsmerkmale der Medikamente zu analysieren, wobei die Medikamente derart angeordnet sind, dass sie sich in jedem der Fächer des zweiten Verteilers nicht überlappen: dass eins der Medikamente, das in einem Fach vorhanden ist, durch einen Raum von einem oder mehreren benachbarten Medikamenten getrennt ist oder dass es mit einem oder mehreren benachbarten Medikament(en) in Kontakt ist;
- Übertragen, durch eine Übertragungsvorrichtung, der analysierten oder nicht analysierten Identifikationsmerkmale, die von der Messvorrichtung ermittelt wurden und sich auf die Medikamente beziehen, die in den Fächern des zweiten Verteilers vorhanden sind, an eine Recheneinheit, wobei die Recheneinheit ausgelegt ist, um:
- die erhaltenen Identifikationsmerkmale für jedes der Medikamente, die in einem Fach des zweiten Verteilers vorhanden sind, zu isolieren und jedes dieser Medikamente zu identifizieren,
- die isolierten Identifikationsmerkmale mit Informationen zu vergleichen, die in einer Datenbank der Merkmale der Medikamente enthalten sind, die gemäß der Verschreibung in einem der Fächer des zweiten Verteilers vorhanden sein müssen, und
- Senden einer Nachricht, gemäß der:
- alle Medikamente, die tatsächlich in jedem der Fächer des zweiten Verteilers vorhanden sind, denen entsprechen oder nicht, die sich gemäß der Verschreibung dort befinden müssten;
- die Anzahl an Medikamenten, die tatsächlich in einem Fach vorhanden sind, der Anzahl an Medikamenten entspricht, die sich gemäß der Verschreibung dort befinden müssten;
- die Anzahl insgesamt an Medikamenten, die in allen Fächern des zweiten Verteilers vorhanden sind, der Anzahl insgesamt an Medikamenten entspricht, die sich gemäß der Verschreibung im zweiten Verteiler befinden müssten;
- das Gewicht der Medikamente, die in einem Fach vorhanden sind, dem Gewicht der Medikamente entspricht, die sich gemäß der Verschreibung dort befinden müssten; und/oder
- das Gewicht aller Medikamente, die in allen Fächern des Verteilers vorhanden sind, dem Gewicht insgesamt aller Medikamente entspricht, die sich gemäß der Verschreibung dort befinden müssten;
- Korrigieren, eventuell, des Inhalts eines der Fächer des zweiten Verteilers bei festgestellter Nicht-Konformität in Bezug auf die medizinische Verschreibung; und
- Transit der in dem Fach des zweiten Verteilers vorhandenen Medikamente in das Fach eines ersten Verteilers gegenüber, wobei der zweiten Verteiler in Bezug auf den ersten Verteiler überdimensioniert ist, anhand einer Verbindungsvorrichtung zwischen den Fächern des ersten Verteilers und denen des zweiten Verteilers.

7. Methode nach Anspruch 6, wobei das Medikament vom Typ Wabenblisterkarte ist, wobei der erste und zweite Verteiler jeweils n Fächer umfassen, wobei n eine Ganzzahl größer oder gleich 7 ist, wobei die Methode den Schritt umfasst
- Senden einer Nachricht bezüglich einer festgestellten Konformität- oder Nicht-Konformität in Bezug auf die medizinische Verschreibung.

8. Methode nach Anspruch 7, wobei die Bestimmung des Inhalts jedes der Fächer je Sektor des Behältnisses erfolgt.

9. Methode nach Anspruch 7 oder 8, wobei die Bestimmung des Inhalts jedes der Fächer anhand der Umsetzung von einem der Etagensysteme zur Identifizierung und Validierung von Medikamenten erfolgt, die in einem der Ansprüche 1 bis 4 definiert sind, und die entsprechenden Messungen mit dem mindestens teilweisen Zusammenlegen von einem Sektor des Behältnisses durchgeführt werden.

10. System nach einem der Ansprüche 1 bis 4, wobei die Freigabevorrichtung eine Gleitplatte als Bestandteil des Bodens der Fächer des zweiten Verteilers ist, wobei sich die Gleitplatte horizontal in parallelen Rillen verlagern kann, die sich in der Verlängerung der Seitenwände von 2 befinden.

11. System nach einem der Ansprüche 2 bis 4 und 10, wobei die Leitungen des Leitungsbündels aus einem elastischen und/oder verformbaren Material sind, das vorzugsweise ein mindestens teilweise transparentes Material ist.

12. System nach einem der Ansprüche 1 bis 4, 10 und 11, wobei die Größe des zweiten Verteilers 1,5 bis 3,5 Mal der Größe des ersten Verteilers entspricht.

13. System nach einem der Ansprüche 1 bis 4 und 10 bis 12, wobei der Abstand zwischen dem unteren Teil des zweiten Verteilers und dem oberen Teil des ersten Verteilers zwischen 10 und 60 cm liegt.

14. Verwendung des Systems nach einem der Ansprüche 1 bis 4 und 10 bis 13 zur Reduzierung des Fehlerrisikos beim Befüllen eines Medikamentenverteilers gemäß einer Verschreibung.

## Claims

1. A stage system for identifying and validating drugs disposed, according to a prescription, in the compartments of a dispenser for a patient and/or for a person accompanying the patient, said system comprising:
- a first dispenser (110) for the patient and/or for a person accompanying the patient;
- a second dispenser (126) oversized compared to the size of the first dispenser, including the same number of compartments or a number of compartments greater than the number of compartments of the first dispenser;
- a connection device (132) between the compartments of the first dispenser and those of the second dispenser, said connection device allowing the transit of one or several drugs, from one compartment of the second dispenser towards its opposite of the first dispenser, the lower part of a compartment of the second dispenser being configured so as to have a releasing device letting, on request, the drugs of one of the compartments of the second dispenser migrate to the opposite compartment of the first dispenser;
- a measuring device (105) configured to collect and analyze drug identification characteristics, the drugs being disposed so as not to overlap, in each of the compartments of the second dispenser: whether one of the drugs present in one compartment is separated by a space from one or several neighboring drugs or whether it is in contact with one or several adjacent drug(s);
- a transmission device configured to transfer the analyzed or non-analyzed identification characteristics collected by the measuring device and relating to the drugs present in the compartments of the second dispenser, to a computation unit; and
- the computation unit being configured to:
- isolate the identification characteristics received from the transmission device for each of the drugs actually present in one compartment of the second dispenser, and identify each of these drugs,
- compare the identification characteristics isolated in the previous step with the information contained in a database of the characteristics of the drugs having to be present, according to the prescription, in one of the compartments of the second dispenser, and
said identification and validation system being configured to emit a message according to which :
- all the drugs actually present in each of the compartments of the second dispenser correspond or not to those that, according to the prescription, should be there;
- the number of drugs actually present in one compartment corresponds to the number of drugs that, according to the prescription, should be there;
- the total number of drugs present in all the compartments of the second dispenser corresponds to the total number of drugs that, according to the prescription, should be in the second dispenser;
- the weight of the drugs present in one compartment corresponds to the weight of the drugs that, according to the prescription, should be there; and/or
- the weight of all the drugs present in all the compartments of the second dispenser corresponds to the total weight of all the drugs that, according to the prescription, should be there.

2. The system according to claim 1, said system having at least one of the following characteristics:
- the drugs are at least partially superimposed and/or alongside at least another drug present in the same compartment of the second dispenser;
- said second dispenser being of sufficient size to avoid the overlapping of drugs;
- said second dispenser being positioned at a greater height than the first dispenser;
- said second dispenser being positioned and advantageously above and/or offset;
- said connection device allowing transit by gravity of one or several drugs from one compartment of the second dispenser towards its opposite of the first dispenser;
- the connection device consisting of a bundle of conduits that are preferably substantially parallel; and
- the upper opening of a conduit of the connection device being larger than the lower orifice of the connection conduit.

3. The system, according to claim 1 or 2, wherein the first and the second dispenser comprise the same even number of compartments.

4. The system according to claim 3, wherein the first and the second dispenser comprise an even number of compartments varying from 7 to 28.

5. A method for manufacturing the system, according to any one of claims 1 to 4, by implementation of at least one of the known assembly techniques such as in particular: screwing, bonding, bolting, casing, riveting and welding.

6. The method for validating the compliance with a medical prescription, the filling of a drug dispenser integrated into a compliance monitoring aid equipment, said method comprising the following steps:
- filling one or several drugs in compartments of a second dispenser, according to a medical prescription;
- determining the content of each of the compartments of the second dispenser by means of a measuring device configured to analyze drug identification characteristics, the drugs being disposed so that they do not overlap in each of the compartments of the second dispenser: whether one of the drugs present in one compartment is separated by a space from one or several neighboring drugs or whether it is in contact with one or several adjacent drugs;
- transmitting, by means of a transmission device, the analyzed or non-analyzed identification characteristics collected by the measuring device and relating to the drugs present in the compartments of the second dispenser, to a computation unit, the computation unit being configured to:
- isolate the identification characteristics received for each of the drugs present in one compartment of the second dispenser, and identify each of these drugs,
- compare the isolated identification characteristics with information contained in a database of the characteristics of the drugs having to be present, according to the prescription, in one of the compartments of the second dispenser,
- emitting a message according to which:
- all the drugs actually present in each of the compartments of the second dispenser correspond or not to those that, according to the prescription, should to be there;
- the number of drugs actually present in one compartment corresponds to the number of drugs that, according to the prescription, should be there;
- the total number of drugs present in all the compartments of the second dispenser corresponds to the total number of drugs that, according to the prescription, should be in the second dispenser;
- the weight of the drugs present in one compartment corresponds to the weight of the drugs that, according to the prescription, should be there; and/or
- the weight of all the drugs present in all the compartments of the dispenser corresponds to the total weight of all the drugs that, according to the prescription, should be there;
- possibly correcting the content of one of the compartments of the second dispenser in the event of non-compliance observed relative to the medical prescription; and
- transiting drugs present in the compartment of the second dispenser into the compartment of a first opposite dispenser, the second dispenser being oversized compared to the first dispenser, by means of a connection device between the compartments of the first dispenser and those of the second dispenser.

7. The method according to claim 6, wherein the drug is of the blister card type, said first and second dispensers each comprising n compartments, n being an integer greater than or equal to 7, and said method comprising the step of:
- sending a message of compliance or non-compliance observed relative to the medical prescription.

8. The method according to claim 7, wherein the determination of the content of each of the compartments is done by vessel sector.

9. The method according to claim 7 or 8, wherein the determination of the content of each of the compartments is done through the implementation of one of the identification and validation stage systems defined in any one of claims 1 to 4, and the corresponding measurements are made with at least partial folding of a sector of the container.

10. The system, according to any one of claims 1 to 4, wherein the releasing device is a sliding plate constituting the floor of the compartments of the second dispenser, the sliding plate can move horizontally in parallel grooves located in the extension of the side walls by 2.

11. The system according to any one of claims 2 to 4 and 10, wherein the conduits of the bundle of conduits are made of a flexible and/or deformable material which is preferably an at least partially transparent material.

12. The system, according to any one of claims 1 to 4, 10 and 11, wherein the size of the second dispenser is 1.5 to 3.5 times the size of the first dispenser.

13. The system according to any one of claims 1 to 4 and 10 to 12, wherein the distance between the lower part of the second dispenser and the upper part of the first dispenser is comprised between 10 and 60 cm.

14. A use of the system, as defined in any one of claims 1 to 4 and 10 to 13, for reducing the risk of error when filling a dispenser with drugs according to a prescription.
